# EUROPEAN PATENT APPLICATION

(11) **EP 2 687 604 A2**
(43) Date of publication of application: **22.01.2014**
(21) Application number: 12197359.8
(22) Date of filing: 18.01.2008
(51) Int. Cl.: C12N 15/82, C12N 15/85, C12N 15/11, C12N 15/113

(54) **Methods and compositions for modulating the miRNA pathway**

(30) Priority: 18.01.2007 US 881433 P; 18.01.2007 US 881434 P
(62) Divisional of application: 08750854.5
(71) Applicant: Plant Bioscience Limited (PBL), Norwich Norfolk NR4 7UH (GB); Navarro, Lionel, 67084 Strasbourg Cedex (FR); Voinnet, Olivier, 67084 Strasbourg Cedex (FR)
(72) Inventor: Navarro, Lionel, 67084 Strasbourg Cedex (FR); Voinnet, Olivier, 67084 Strasbourg (FR)
(74) Representative: Novagraaf Technologies

(57) **Abstract**

A specific spectrum of plant and animal miRNAs confer enhanced resistance to virulent pathogens. Plants deficient in miRNA accumulation are hyper-susceptibble to non-virulent bacterial pathogens, and virulent bacteria suppress miRNA pathways by means of injected type-III secreted (TTS) proteins. Methods and compositions for modulating the miRNA pathway in plants and animals are disclosed whereby adverse effects on plant and animal development are avoided or minimized.

## Description

### RELATED APPLICATIONS

This application claims benefit of U.S. application(s) Serial Numbers 60/881,443 and 60/881,434 both filed 18 January 2007 which are incorporated herein by reference in their entirety.

### FIELD OF THE INVENTION

Compositions and methods for conferring broad spectrum pathogen resistance, against plant and animal pathogens.

### BACKGROUND OF THE INVENTION

In recent years, there has been an ever increasing appreciation of the complexity and pleiotropic effects of gene silencing and components of the gene silencing machinery. From effects observed initially via transgene suppression of endogenous gene expression in petunia plants, has emerged an understanding of a penumbra of effects in plants and animals spanning antiviral defense and control of transposons through RNA-directed DNA methylation.

### Small RNA, Dicers and Argonautes: the biochemical core of RNA silencing

"RNA silencing" refers collectively to diverse RNA-based processes that all result in sequence-specific inhibition of gene expression, either at the transcriptional, mRNA stability or translational levels. Those processes share three biochemical features: (i) formation of double-stranded (ds)RNA, (ii) processing of dsRNA to small (s) 20-26nt dsRNAs with staggered ends, and (iii) inhibitory action of a selected sRNA strand within effector complexes acting on partially or fully complementary RNA/DNA. While several mechanisms can generate dsRNA, the sRNA processing and effector steps have a common biochemical core. sRNAs are produced by RNAseIII-type enzymes called Dicers¹ with distinctive dsRNA binding, RNA helicase, RNAseIII and PAZ (Piwi/Argonaute/Zwille) domains. One of the two sRNA strands joins effector complexes called RISCs (RNA-induced silencing complex) that invariably contain a member of the Argonaute (Ago) protein family. Agos have an sRNA binding PAZ domain and also contain a PIWI domain providing endonucleolytic ('slicer') activity to those RISCs programmed to cleave target RNAs^{2,3}. In fact, sRNA-loaded human Ago2 alone constitutes a cleavage-competent RISC *in vitro,* but many additional proteins may be functional components of RISCs *in vivo*⁴*.*

Here, we review recent evidence that several pathways built over the Dicer-Ago core execute a diverse set of sRNA-directed biological functions in higher plants. These include regulation of endogenous gene expression, transposon taming, viral defense and heterochromatin formation. Our focus is primarily on plants because they exhibit a nearly full spectrum of known RNA silencing effects, but similarities and differences with other organisms are also discussed.

### Exogenously triggered RNA silencing pathways resulting in transcript cleavage dsRNA-producing transgenes and IR-PTGS: useful, but mysterious

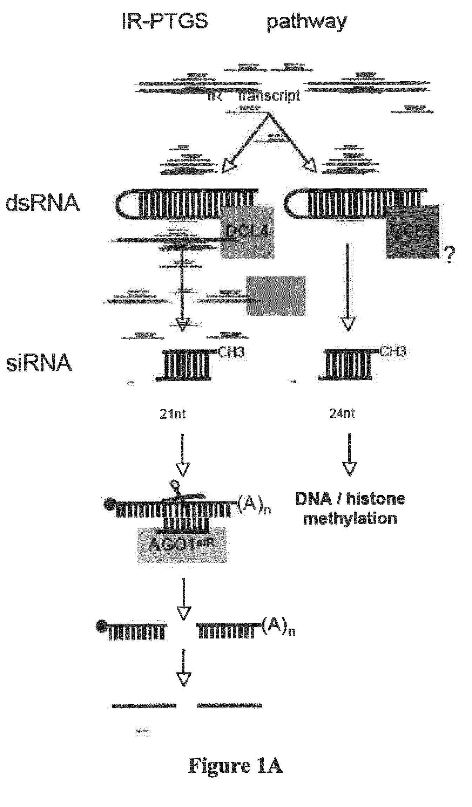

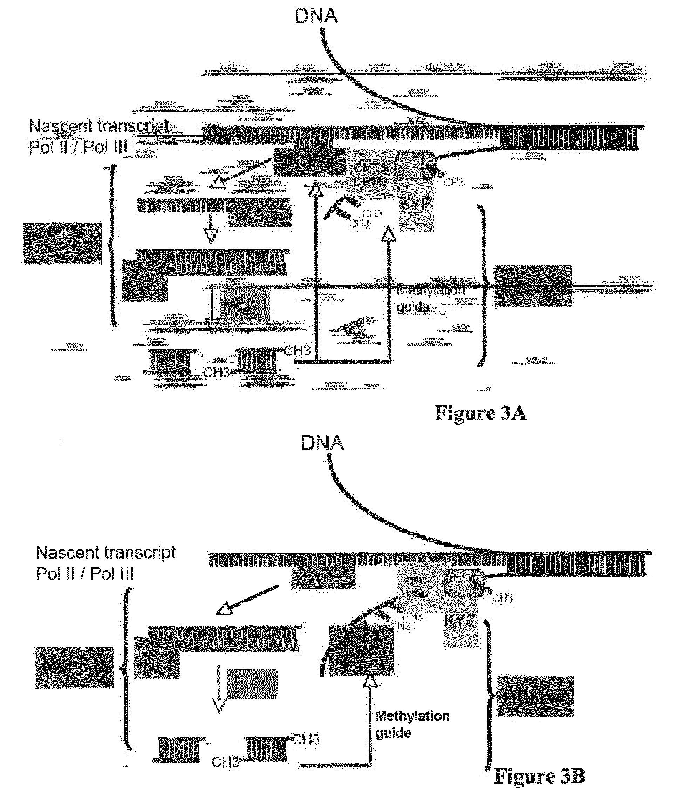

Post-transcriptional gene silencing (PTGS) was discovered in transgenic *Petunia* as loss of both transgene (in either sense or antisense configuration) and homologous endogenous gene expression⁵. The transgene loci often produced dsRNA because they formed arrays with complex integration patterns^{6,7}. Accordingly, PTGS efficacy was greatly enhanced by simultaneous sense and antisense expression⁸ or by direct production of long dsRNA from inverted-repeat (IR) transgenes⁹. The latter process, IR-PTGS, currently forms the basis of experimental RNAi in plants, and involves at least two distinct sRNA classes termed short interfering (si)RNAs. 21nt siRNAs are believed to guide mRNA cleavage, while 24nt siRNAs may exclusively mediate chromatin modifications^{10, 11}. Both siRNA classes accumulate as populations along the entire sequence of IR transcripts. Although widely used as a research tool, IR-PTGS remains one of the least understood plant RNA silencing processes (Figure 1A). Figure 1A shows how an inverted repeat (IR) transgene construct, typically employed for RNAi in plants, produces double-stranded (ds) transcripts with perfectly complementary arms. Two distinct Dicer-like (DCL) enzymes process the ds transcripts. DCL3 most likely produces siRNAs of the 24nt size class, which may direct DNA/histone modification at homologous loci (see Figure 3) and appear dispensable for RNA cleavage. DCL4 is likely the preferred enzyme for production of 21nt-long siRNAs from the dsRNA. One siRNA strand incorporates into AGO1-loaded RISC to guide endonucleolytic cleavage of homologous RNA, leading to its degradation. Both siRNA species are protected from degradation by addition of methyl groups at the 3' termini of each RNA strand, by the methyl-transferase HEN1.

Hence, until recently, no mutant defective in this pathway had been recovered, despite considerable efforts in several laboratories. One likely explanation is that the high dsRNA levels produced in IR-PTGS promote the activities of different Dicers and RISCs, which would normally act in distinct pathways, to redundantly mediate silencing. Recent analyses of combinatorial Dicer knockouts in *Arabidopsis* support this idea^{12, 13}. Nonetheless, Dicer-like 4 (DCL4) seems a preferred enzyme for IR-PTGS because it was specifically required for 21nt siRNA accumulation and silencing from a moderately expressed, phloem-specific IR transgene¹⁴. DCL2 might also be involved in RNAi, because it processes some endogenous DCL4 substrates into 22nt-long siRNAs in the absence of DCL4^{12, 13}, although it remains unclear if those molecules can functionally substitute for the 21nt siRNA products of DCL4.

### S-PTGS and transitive silencing: enter RDR

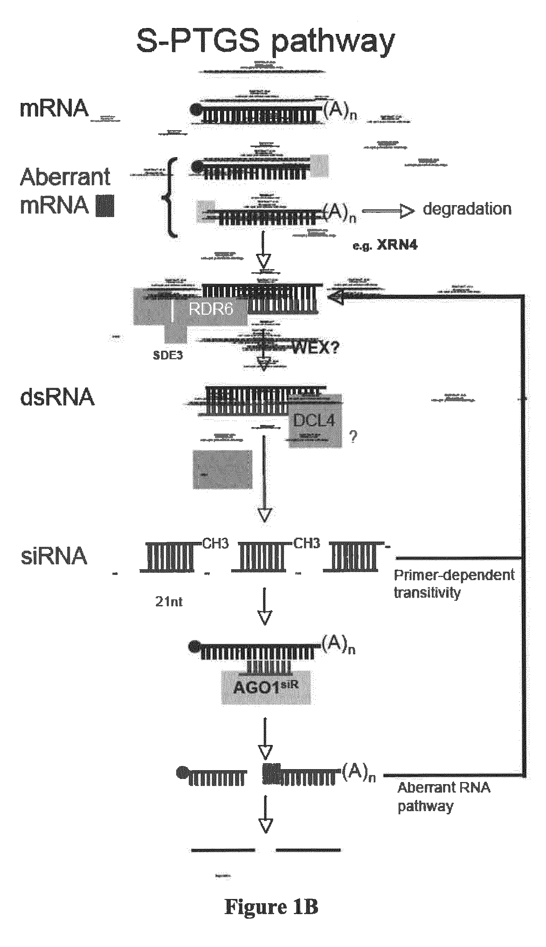

There are several examples in which single-copy transgene insertions producing sense transcripts trigger PTGS. This pathway, sense (S)-PTGS, has been dissected using *Arabidopsis* forward-genetic screens that provided insights into how dsRNA is produced (Figure 1B). These screens converged on the identification of the RNA-dependent RNA polymerase RDR6, one of six putative *Arabidopsis* RDRs^{15, 16}. RDR6 is thought to recognize and to use as templates certain transgene transcripts with aberrant features that include lack of 5' capping. For instance, mutation of *Arabidopsis* XRN4, a 5'-3' exonuclease that degrades uncapped mRNAs, enhanced accumulation of uncapped transgene mRNAs. This favored their conversion into dsRNA by RDR6 and the subsequent degradation of all transgene transcripts through the S-PTGS pathway¹⁷. RDR6 most likely synthesizes complementary strands from its RNA templates, resulting in dsRNA production, because a missense mutation in the GDD motif, essential for the catalytic activity of all characterized RDRs, is sufficient to alleviate S-PTGS¹⁶.

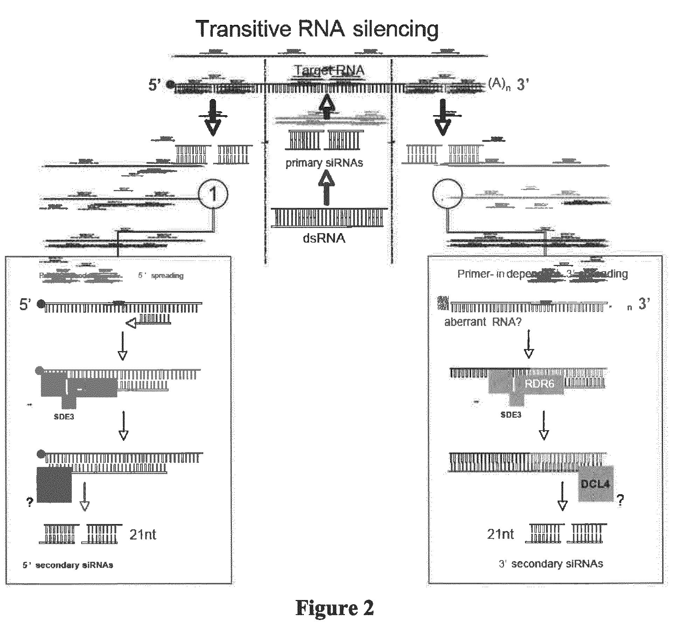

Although the Dicer producing siRNAs from RDR6 products remains to be formally identified, S-PTGS siRNA accumulation in *Arabidopsis* requires the coiled-coil protein of unknown function SGS3¹⁶, the RNAseD exonuclease WEX¹⁸, the sRNA-specific methyl transferase HEN1¹⁹ and the putative RNA helicase SDE3²⁰ (Figure 1B). In Figure 1B, the pathway is shown as being elicited by RNAs with aberrant features, although there might be alternative triggers. The RNA aberrations could include lack of a poly-A tail or lack of 5' capping. The latter would normally lead to RNA degradation through the activity of the 5'-3' exonuclease XRN4. Lack of XRN4 would promote accumulation of uncapped mRNA, thereby triggering their conversion into dsRNA by the combined action of RDR6, SGS3, SDE3 and, possibly, WEX. The resulting dsRNA is then processed by a DCL, most likely DCL4 (see text), producing siRNAs that are exclusively of the 21nt size class and methylated by HEN1. These molecules can engage into two sets of reactions. First, they can be used as primers by RDR6 to reinforce production of dsRNA from single-stranded templates through a phenomenon known as 'transitivity' (see Figure 2). They can also incorporate into AGO1-loaded RISC to guide sequence-specific cleavage of homologous RNA. The resulting cleavage products could be perceived as aberrant RNAs and, thus, could promote further production of dsRNA, resulting in an amplified reaction.

Unlike RDR6, SDE3 is not stringently required for transgene silencing, and so could accessorily resolve the secondary structures found in RDR templates²⁰. Accordingly, an SDE3 homologue is part of the *Schizosaccharomyces pombe* RDR complex²¹. SDE3 could also act at other RNA silencing steps because the homologous protein Armitage is required for RISC assembly in *Drosophila,* an organism deprived of *RDR* genes²². WEX is related to the exonuclease domain of *mut-7,* required for transposon silencing and RNAi in *C*. *elegans* but its role in S-PTGS remains elusive²³. HEN1-catalyzed methylation of free hydroxy termini protects *Arabidopsis* sRNAs, including S-PTGS siRNAs, from oligo-uridylation, a modification promoting their instability (see the miRNA section of the background below)²⁴.

In one S-PTGS mutant screen, an extensive allelic series of *ago1* was recovered, arguing that among the 10 *Arabidopsis AGO* paralogs, AGO1 is specifically involved in this pathway^{25, 26}. Even weak *ago1* alleles completely lost S-PTGS siRNAs, initially suggesting a role for AGO1 in siRNA production rather than action²⁶. However, since AGO1 is now recognized as a slicer activity of the plant miRNA- and siRNA-loaded RISCs, loss of siRNAs in *ago1* may also result from their poor incorporation into RISC, enhancing their turnover. Nevertheless, a role for AGO1 in siRNA production - possibly linked to RDR6-dependent dsRNA synthesis - cannot be excluded because some *ago1* mutants defective in S-PTGS siRNA accumulation show no defects in IR-PTGS²⁷_{.}

RDR6, and perhaps other S-PTGS components, is also involved in the related silencing phenomenon, transitivity^{28, 29}. Transitivity is the "transition" of primary siRNAs (corresponding to a sequence interval of a targeted RNA) to secondary siRNAs targeting regions outside the initial interval (Figure 2). Figure 2 shows how in transitive RNA silencing, a dsRNA source of primary siRNAs promotes production of secondary siRNAs both 5' and 3' of the initially targeted interval of a transcript. Production of 5' secondary siRNAs (case 1) can be explained by RDR6/SGS3/SDE3-dependent complementary strand synthesis that is primed by one of the primary siRNAs. Production of 3' secondary siRNAs (case 2) cannot be explained by a primed reaction, and it is possible that RNA fragments resulting from primary siRNA-directed transcript cleavage are recognized as aberrant, thereby initiating dsRNA synthesis as in S-PTGS. The 5' and 3' reactions should not be considered mutually exclusive, as siRNAs produced in (2) could prime further dsRNA synthesis according to the scheme depicted in (1). DCL4 is shown as putatively involved in 5' and 3' secondary siRNA biogenesis. Unlike primary siRNAs (which can be 21nt and 24nt in size), secondary siRNA are exclusively of the 21nt size class. It remains unclear whether 24nt primary siRNAs can trigger transitive RNA silencing.

In plants, this transition may occur both 5' and 3' to the primary interval, possibly reflecting primer-dependent and primer-independent RDR6 activities. Transitivity serves as a siRNA amplification mechanism that also accounts for extensive movement of silencing throughout transgenic plants³⁰. Secondary siRNAs are exclusively of the 21nt size class³⁰. Thus, given that S-PTGS siRNAs seem to accumulate as 21nt species³², that DCL4 produces the 21nt siRNAs from IR transcripts¹⁴, and that DCL4 and RDR6 activities are coupled for 21nt *trans-acting* siRNA biogenesis (see below), it is tempting to speculate that DCL4 is also the preferred Dicer for siRNA production in both S-PTGS and transitivity (Figure 1B, 2).

What would be the biological function of an amplified and non-cell autonomous pathway based on 21nt siRNAs? At least one answer is antiviral defense. Virus-derived 21nt siRNAs accumulate in infected cells³¹ and plants compromised for RDR6 function are hypersusceptible to several viruses^{16, 32}. An RDR-amplified response primed by viral siRNAs (transitivity) and/or elicited by viral-derived aberrant RNAs (S-PTGS pathway) would ensure that the silencing machinery keeps pace with the pathogen's high replication rates. The systemic nature of the response would immunize cells that are about to be infected, resulting, in some cases, in viral exclusion. Consistent with this idea, the meristems of *Nicotiana benthamiana* with compromised RDR6 activity became invaded by several viruses, whereas those tissues are normally immune to infection³³.

### Endogenous RNA silencing pathways involved in post-transcriptional regulation

### MicroRNAs

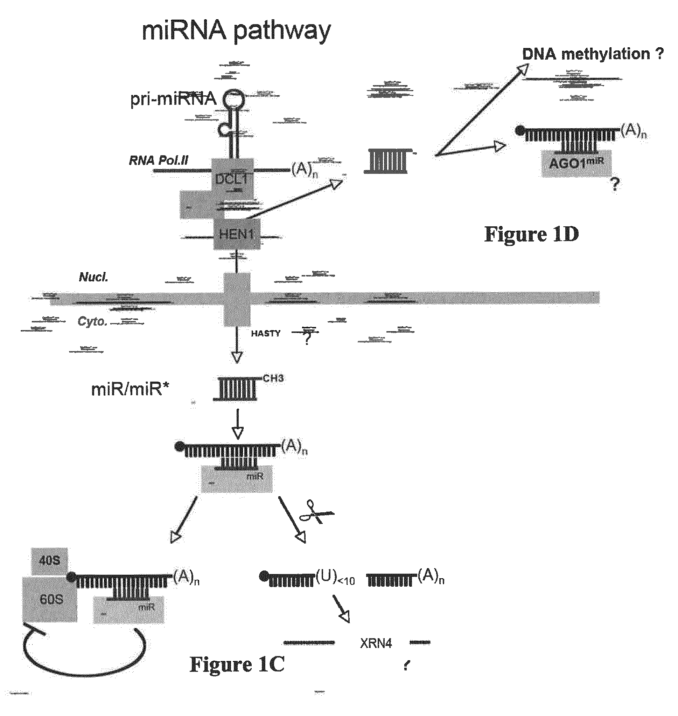

In plants, miRNAs are produced as single-stranded, 20-24nt sRNA species, excised from endogenous non-coding transcripts with extensive fold-back structure. miRNAs act in *trans* on cellular target transcripts to induce their degradation *via* cleavage, or to attenuate protein production (Figure 1C)³⁴. Figure 1C shows how primary (pri) miRNA transcripts with fold-back structures are products of RNA polymerase II (Pol II). The position of the mature miRNA is boxed. The combined nuclear action of DCL1, HYL1 and HEN1 produces a mature, methylated miRNA. Upon nuclear export, possibly mediated by the *Arabidopsis* exportin 5 homolog HASTY, the mature miRNA incorporates into AGO1-loaded RISC to promote two possible sets of reactions that are not mutually exclusive. A first reaction would lead to endonucleolytic cleavage of homologous RNA, as directed by 21nt siRNAs. This would result in a poly-urydilated 5' cleavage fragment -a modification that might promote its rapid turnover- and a more stable 3' fragment that could be degraded by the XRN4 exonuclease. The scheme also accommodates the possibility that mature miRNAs could have sequence-specific effects in the nucleus (see text). Those nuclear activities include RNA cleavage (upon incorporation into a putative nuclear RISC) as well as DNA methylation.

Currently, approximately 100 *Arabidopsis MIRNA* genes falling into 25 distinct families have been identified³⁵, but many more are likely to exist. miRNAs have important biological roles in plant and animal development, as evidenced by the strong developmental defects of several miRNA overexpression and loss-of-function mutants³⁴. For instance, key regulatory elements of the plant response to the hormone auxin, which specifies organ shape and the axes of the plant body, are controlled by miRNAs^{36, 37}. miRNAs also regulate accumulation of transcription factors (TFs) involved in floral organ identity/number^{38, 39}, leaf shape⁴⁰, abaxial/adaxial leaf asymmetry^{41, 42}, and lateral root formation⁴³. In addition, DCL1 and AGO1, involved in the miRNA pathway, are themselves regulated by miRNAs^{44, 45}. Nonetheless, plant miRNAs with validated targets involved in primary and secondary metabolism have been identified^{36, 46}, indicating that their roles are not confined to developmental regulations. miRNAs might, indeed, have broad implications in plant physiology and environmental adaptation.

### miRNA transcription and biogenesis

Most plant and animal miRNA genes reside between protein coding genes or within introns⁴⁷. Most are likely to be independent transcription units and their expression patterns often show exquisite tissue- or even cell-type specificity, in agreement with a role in patterning and maintenance of differentiated cell states^{48, 49}. Nonetheless, transcription factors or post-transcriptional mechanisms that specify plant miRNA gene expression remain unknown. Many human primary miRNA transcripts (pri-miRNAs) are synthesized by RNA polymerase II (Pol II), because pri-miRNAs have typical Pol II 5' caps and poly-A tails, their synthesis is inhibited by PolII-inhibiting drugs, and PolII is found at their promoters *in vivo*⁵⁰ Similar, though less extensive, evidence also points to PolII as the major polymerase producing plant pri-miRNAs³⁵.

Upon transcription, mammalian pri-miRNAs are processed *via* a well-defined biosynthetic pathway. The RNAseIII Drosha and its essential cofactor DGCRB/Pasha- both constituents of the nuclear Microprocessor complex - catalyze initial cuts at the basis of pri-miRNAs stem-loop to produce pre-miRNAs. Pre-miRNAs are processed by Dicer into mature miRNAs upon Exportin-5-dependent nuclear export⁵¹. Plants have no direct equivalent of Microprocessor. In *Arabidopsis,* miRNA biosynthesis depends specifically upon DCL1^{52, 53}, which is required for the nuclear stepwise processing of pri-miRNAs. Whether DCL1 itself catalyzes all of the reactions involved is uncertain⁵⁴. The plant exportin-5 homolog HASTY is involved in miRNA biogenesis⁵⁵, but its exact role is not as clear as in mammals where the Microprocessor pre-miRNA product is an experimentally verified cargo⁵⁶. *hasty* mutants exhibit decreased accumulation of some, albeit not all, miRNAs in both nuclear and cytoplasmic fractions⁵⁵. These observations support the existence of HASTY-independent miRNA export systems and question whether miRNAs or miRNA-containing complexes are even direct cargoes of HASTY.

In plants and animals, Dicer processing occurs in association with specific dsRNA-binding proteins. First observed with the Dcr2-R2D2 complex required for RISC loading in the *Drosophila* RNAi pathway⁵⁷, this has now also been found for the Dcr1-Loqs complex involved in the *Drosophila* miRNA pathway⁵⁸, and Dicer-TRBP as well as Dicer-PACT in human cells^{59, 60}. DCL1-HYL1 constitutes a similar complex that acts in pri-miRNA processing in the *Arabidopsis* miRNA pathway (Figure 1C).⁶¹⁻⁶⁴ In all cases, Dicer produces a duplex between the mature miRNA (miR) and its complementary strand (miR*)⁶⁵. The miR strand is generally least stably base-paired at its 5'-end and is, consequently, loaded as the guide strand into RISC, whereas the miR* strand is degraded⁶⁶ (Figure 1C). In the *Drosophila* RNAi pathway, R2D2 acts as a thermodynamic asymmetry sensor of siRNA duplexes, and Loqs, TRBP, PACT and HYL1 could possibly perform similar roles.

HEN1 is an S-adenosyl methionine (SAM)-binding methyl transferase that methylates the 2' hydroxy termini of miR/miR* duplexes, a reaction apparently specific to the plant kingdom⁶⁷, ⁶⁸. Methylation protects miRNAs from activities that uridylate and degrade plant sRNAs from the 3'end^{24,} but it is not required for RISC-dependent miRNA-guided cleavage in *Arabidopsis* extracts{Qi, 2005 #5064}. All known classes of plant sRNAs are methylated by HEN1²⁴, but this modification seems to impact differentially on sRNA stability, perhaps reflecting variable interactions between HEN1 and distinct protein complexes or distinct sRNA populations. For example, the viral silencing suppressor Hc-Pro prevents methylation of virus derived siRNAs, but not of miRNAs ⁶⁹and several *hen1* mutant alleles exist, in which accumulation of miRNA, but not of S-PTGS siRNAs, is impaired ¹⁹.

### Plant miRNA activities

Most identified plant miRNAs have near-perfect complementarity to their targets and promote their cleavage. This is followed by oligo-uridylation and rapid degradation of the 5'-cleavage fragment⁷⁰, and slower degradation of the 3'-cleavage fragment mediated, at least in some cases, by XRN4⁷¹ (Figure 1C). Animal miRNAs generally exhibit imperfect complementarity and repress protein production from intact target mRNAs. However, it is possible that the action of both plant and animal miRNAs results from a combination of both processes, whose respective contributions probably vary depending on the extent of the miRNA:target complementarity. Although the RISC(s) acting in the plant miRNA pathway remain ill defined, AGO1 associates with miRNAs and miRNA targets are cleaved *in vitro* by immuno-affinity-purified AGO1. Thus, in plants, the same Argonaute appears to function as a Slicer for both miRNA- and siRNA-loaded RISCs, contrasting with the situations in *Drosophila* and *C. elegans.* Plant RISC components other than AGO1 await identification and it may well be that several alternative RISCs exist, given the number of *AGO*-like genes in *Arabidopsis.*

Mature plant miRNAs are detected in both nuclear and cytosolic cell fractions⁵⁵. Likewise, RISC programmed with the *let-7* miRNA can be immuno-purified from nuclear human cell fractions⁷², indicating that plant and animal miRNAs may have nuclear functions (Figure 1C). These may include RNA cleavage, as suggested by the intron-targeting activity of the plant miR173⁷³, but could also comprise modifications of homologous DNA⁷⁴. Thus, in *Arabidopsis,* miR165 recognition of the spliced *PHB* transcript apparently directs *cis-*methylation on the *PHB* template DNA. This methylation is enigmatic, however, as it occurs several kb downstream of the miRNA binding site⁷⁴. It is conceivable that miRNA-induced cleavage of the nascent *PHB* transcript triggers dsRNA formation initiated at the 3'end of the transcript through a primer-independent RDR activity with moderate processivity. The resulting production of siRNA would thus be confined to the 3'end and could mediate DNA methylation according to the schemes discussed in a further section of this review. Intriguingly, some, albeit few, siRNAs corresponding to downstream parts of several miRNA targets have been detected in *Arabidopsis,* although none were directly complementary to the methylated *PHB* sequence⁷⁵. Direct miRNA-guided DNA methylation in *cis* and/or *trans* has also been suggested from the observation that some 21nt miRNAs of *Arabidopsis* accumulate as a second, 24nt species at specific developmental stages⁶⁵.

### Transacting siRNAs: mixing up miRNA and siRNA actions

Transacting (ta) siRNAs are a recently discovered class of plant endogenous sRNAs. They derive from non-coding, single-stranded transcripts, the pri-tasiRNAs, which are converted into dsRNA by RDR6-SGS3, giving rise to siRNAs produced as discrete species in a specific 21nt phase^{76, 77} (Figure 1D). Figure 1D shows how primary (pri) trans-acting siRNA transcripts are non-coding RNAs devoid of extensive fold-back structures. A miRNA incorporated into AGO1-loaded RISC guides endonucleoytic cleavage of the pri-tasiRNA. This cut generates two cleavage fragments, one of which acts as an RDR6 template, leading to the production of dsRNA. DCL4 initiates processing exclusively from the dsRNA ends corresponding to the initial miRNA cut site, to produce phased tasiRNAs that are methylated by HEN1. tasiRNA subsequently guide cleavage of homologous mRNAs, once incorporated into AGO1-loaded RISC. The colored reactions depicted in the inlay illustrate the importance of the initial miRNA-directed cut in determining the appropriate phase for tasiRNAs (1). Incorrect phasing (2) would result in the production of off-target small RNAs.

The RDR6-SGS3 involvement is reminiscent of siRNA biogenesis in S-PTGS, but the genetic requirements of those pathways are not identical, because tasiRNA accumulation is normal in the hypomorphic *ago1-27* mutant and in mutants defective in SDE3 and WEX⁷⁶. Much like plant miRNAs, mature tasiRNAs guide cleavage and degradation of homologous, cellular transcripts. To date, tasiRNA generating loci *(TAS1-3)* have been only identified in *Arabidopsis⁷³,* but they are likely to exist in other plant species and possibly in other organisms that contain RDRs such as *C*. *elegans* or *N. crassa.*

tasiRNA production involves an interesting mix of miRNA action and the siRNA biogenesis machinery. Pri-tasiRNAs contain a binding site for a miRNA that guides cleavage at a defined point. The initial miRNA-guided cut has two important consequences. First, it triggers RDR6-mediated transitivity on the pri-tasiRNA cleavage products, allowing dsRNA production either 5' or 3' of the cleavage site⁷³. Second, it provides a well-defined dsRNA terminus crucial for the accuracy of a phased dicing reaction, performed by DCL4, which produces mature tasiRNAs (Figure 1D).

What is the biological role of tasiRNAs? *rdr6, sgs3,* and *dcl4* all exhibit accelerated juvenile-to-adult phase transition^{12, 13, 77, 78}, indicating that tasiRNAs could regulate this trait. The tasiRNA targets include two auxin response factor (ARF) TFs and a family of pentatricopeptide repeat proteins, although there is no evidence for the involvement of the only functionally characterized target (ARF3/ETTIN) in juvenile-to-adult phase transition⁷⁹, nor were heterochronic defects noticed in insertion mutants disrupting the *TAS1* or *TAS2* loci^{76, 78}. Mutants in *AGO7*/*ZIPPY* display a similar phase transition defect⁸⁰, suggesting that AGO7 could be part of a specific tasiRNA-programmed RISC, although tasiRNAs do co-immunoprecipitate with AGO1 to form a cleavage competent RISC{Qi, 2005 #5064}.

### Natural antisense transcript siRNAs

An example has been recently described in which a pair of neighboring genes on opposite DNA strands (cis-antisense genes) gives rise to a single siRNA species from the overlapping region of their transcripts⁸¹. This 24nt siRNA species - dubbed natural antisense transcript siRNA (nat-siRNA)- guides cleavage of one of the two parent transcripts, and is produced in a unique pathway involving DCL2, RDR6, SGS3 and the atypical DNA dependent RNA polymerase-like subunit NRPD1a (see the discussion of chromatin targeted RNA silencing pathways below). nat-siRNA guided cleavage triggers production of a series of secondary, phased 21nt siRNAs, a reaction similar to tasiRNA biogenesis except that the Dicer involved is DCL1. The role of secondary nat-siRNAs is currently unclear, but primary nat-siRNA-guided cleavage contributes to stress adaptation, and, given the large number of *cis* antisense gene pairs in plant and other genomes ^{82, 83}, this isolated example may reflect a widespread mechanism of gene regulation.

### Chromatin targeted RNA silencing pathways

In addition to acting on RNA, siRNAs can guide formation of transcriptionally silent heterochromatin in fungi, animals and plants. Plant heterochromatin is characterized by two sets of modifications: methylation of cytosines and of specific histone lysine residues (histone 3 Lys9 (H3K9) and histone 3 Lys27 (H3K27) in *Arabidopsis*)⁸⁴*.* In some organisms, these modifications act as assembly platforms for proteins promoting chromatin condensation. *Arabidopsis* cytosine methyl-transferases include the closely homologous DRM1/2 required for all *de novo* DNA methylation, MET1 required for replicative maintenance of methylation at CG sites, and CMT3 required for maintenance at CNG and asymmetrical CNN sites (reviewed in^{85, 86}). Histone methyl-transferases involved in H3K9 and H3K27 methylation belong to the group of Su(Var)3-9 homologues and include KYP/SUVH4 and SUVH2 in *Arabidopsis*⁸⁷*.*

In several organisms, siRNAs corresponding to a number of endogenous silent loci, including retrotransposons, 5S rDNA and centromeric repeats, have been found⁸⁵. They are referred to as cis-acting siRNAs (casiRNAs) because they promote DNA/histone modifications at the loci that generate them. In plants, casiRNAs are methylated by HEN1 and are predominantly 24nt in size. Their accumulation is specifically dependent upon DCL3 and, in many instances, upon RDR2. casiRNA accumulation also requires an isoform (containing subunits NRPD1a and NRPD2) of a plant-specific and putative DNA-dependent RNA polymerase, termed PolIV⁸⁸⁻⁹⁰. PolIV may act as a silencing-specific RNA polymerase that produces transcripts to be converted into siRNAs by the actions of RDR2 and DCL3. However, many aspects of PolIV silencing-related activities remain obscure. Hence, it is uncertain whether PolIV even possesses RNA polymerase activity. Additionally, a distinct PolIV isoform with subunits NRPDlb and NRPD2 is required for methylation directed by IR-derived siRNAs with transgene promoter homology, suggesting that the action of PolIV complexes may not be confined to siRNA biogenesis⁹¹. Finally, the requirement of NRPD1a for nat-siRNA accumulation in the presence of both antisense mRNAs (produced by PolII) suggests that PolIV may have silencing-related functions independent of DNA-dependent RNA polymerase activity⁸¹. Other factors involved in IR-derived siRNA-directed promoter methylation include the chromatin remodelling factor DRD1⁹² and the putative histone deacetylase HDA6⁹³ whose activity may be required to provide free histone lysines for methylation by KYP/SUVH enzymes (Figure 3). It is currently uncertain whether DRD1 and HDA6 are also implicated in silencing of endogenous loci. 24nt siRNAs may act in a RISC-like complex, perhaps akin to the RNA-induced transcriptional silencing complex, RITS, characterized in fission yeast⁹⁴. This complex could contain AGO4 because *ago4* mutants have phenotypes overlapping with those of *rdr2, dcl3, nrpd1a a* and *nrpd2*¹¹*.* At loci affected by the above mutations, CNG and particularly CNN methylation is strongly reduced, whereas loss of CG methylation is less pronounced, consistent with the observation that MET1-dependent promoter CG methylation could be maintained in the absence of a viral-encoded RNA trigger of TGS⁹⁵.

DNA itself or nascent transcripts are both possible targets of casiRNAs (Figure 3A and 3B, respectively). In Figure 3A, nascent polII/polIII transcript is cleaved through the action of siRNA-programmed AGO4, resulting in a truncated RNA that is converted into dsRNA by the action of RDR2. The dsRNA is then processed by DCL3 into 24nt siRNAs that direct further cleavage of nascent transcripts and may possibly guide sequential activities of histone deacetylases (*e.g.,* HDA6), histone methyl transferases (*e.g.,* KYP, SUVH2) and/or DNA methyl-transferases (CMT3/DRM). It is unclear whether histone modification precedes DNA methylation or not. The process might also involve siRNA-directed chromatin remodeling factors such as DRD1. The positions of PolIVa and PolIVb in those reactions are currently ill defined.

In Figure 3B, the same effectors are involved but, in this scenario, RDR2 uses nascent transcripts as templates and siRNA-loaded AGO4 is recruited to guide chromatin modifications rather than RNA cleavage.

In the *S. pombe* heterochromatic RNAi pathway resulting in H3K9 (but not cytosine) methylation, target transcription by PolII is required for siRNA action, and Ago1 associates with nascent transcripts⁹⁶. siRNA directed histone methylation of the human EF1A promoter was also dependent on active PolII transcription⁹⁷. However, direct siRNA-DNA base-pairing cannot be excluded. For instance, in experiments involving virus derived promoter directed siRNAs, the methylated DNA interval on targeted promoters matched the primary siRNA source and did not extend any further into transcribed regions⁹⁵. If siRNAs indeed interact directly with DNA, how does the double helix become available for siRNA pairing? PolIV could facilitate this access, for instance by moving along the DNA with associated helicases. The precise molecular mechanisms underlying sequence-specific recruitment of cytosine and histone methyl-transferases to silent loci also remains elusive, as associations between sRNA and such enzymes have been reported in only one single case, in human cells⁹⁷. In fact, a self-sustaining loop in which siRNA production and DNA/histone methylation are mutually dependent appears to exist at endogenous silent loci, raising the possibility that production of chromatin-directed siRNAs *in vivo* might even be a consequence, rather than a cause, of DNA/histone methylation (Figure 3).

The RDR2/DCL3/NRPD1/AGO4 pathway has clear roles in transposon taming and maintenance of genome integrity in plants, because loss of casiRNA caused by mutations in the above factors reactivates transposon activity. This pathway may also maintain heterochromatin at centromeric repeats, which appears mandatory for accurate chromosome segregation in *S. pombe*⁹⁸*.* The 24nt siRNA-generating machinery may also act to silence protein-coding genes. For example, expression of the key negative regulator of flowering *FLC* is maintained at a low level in an early-flowering *Arabidopsis* ecotype due the presence of an intronic transposon that causes repressive chromatin modifications through the action of an NRPD1a/AGO4-dependent pathway⁹⁹. Nevertheless, several additional mechanisms, not necessarily mediated by siRNAs, account for epigenetic regulation of gene expression in plants. For example, in *Arabidopsis,* mutation of the chromatin-remodeling factor DDM1 has much broader consequences on chromatin silencing than any known single mutant in the RNA silencing machinery^{100, 101}. In addition, gene regulation by polycomb-like proteins in *Arabidopsis* has not been linked to RNA silencing¹⁰².

**Table 1 Overview of proteins with roles in Arabidopsis small RNA pathways.**

| **Protein** | **Domains and motifs** | **Biochemical activity** | **Pathway** | **Ref.** |
|---|---|---|---|---|
| DCL1 | RNase III | miRNA synthesis | miRNA | 55, 85 |
| | dsRNA bd | | nat-siRNA | |
| | DEAD-box helicase | | | |
| | PAZ | | | |
| | DUF283 (unknown function) | | | |
| HYL1 | dsRNA bd | dsRNA bd | miRNA | 61,62 |
| HST | RanGTP bd | Putative exportin | miRNA | 58 |
| AGO1 | PAZ | siRNA Slicer | miRNA | |
| | Piwi | miRNA Slicer | S-PTGS | |
| | | | tasiRNA | |
| | | | Chromatin (?) | |
| HEN1 | dsRNA bd | sRNA methyl transferase | All sRNA | 19, 24, 53, 67 |
| | Lupus La RNA bd | | pathways | |
| | S-adenosyl bd | | | |
| RDR6 | RdRP-specific GDD | RNA-dependent RNA polymerase | S-PTGS Transitivity tasiRNA nat-siRNA | 15, 16, 29, 30, 73, 76, 85 |
| SGS3 | Coiled-coil | Unknown | S-PTGS | 16, 76, 85 |
| | Putative Zn^{II}-bd | | Transitivity | |
| | | | tasiRNA | |
| | | | nat-siRNA | |
| DCL4 | RNase III | 21nt siRNA synthesis | tasiRNA | 12-14 |
| | dsRNA bd | | IR-PTGS | |
| | Helicase | | S-PTGS? | |
| | PAZ | | | |
| WEX | 3'-5' exonuclease | Putative 3'-5' exonuclease | S-PTGS | 18 |
| SDE3 | DEAD box | Putative RNA helicase | S-PTGS | 20,30 |
| | Helicase | | Transitivity | |
| DCL2 | RNaseIII | 22/24 nt siRNA synthesis | nat-siRNA | 85 |
| | dsRNA bd | | | |
| | PAZ | | | |
| DCL3 | RNase III | 24nt siRNA synthesis | Chromatin | |
| | DEAD box helicase | | | |
| | PAZ | | | |
| RDR2 | RdRP | Putative RNA dependent RNA polymerase | Chromatin | |
| AGO4 | PAZ Piwi | Unclear | Chromatin | 11 |
| NRPD1a | RNA polymerase | Putative DNA dependent | Chromatin | 85, 88-91 |
| | | RNA polymerase | nat-siRNA | |
| NRPD1b | RNA polymerase | Putative DNA dependent RNA polymerase | Chromatin | 89,91 |
| NRPD2 | RNA polymerase | Putative DNA dependent RNA polymerase | Chromatin | 88-91 |
| HDA6 | Histone deacetylase | Putative histone deacetylase | Chromatin | 93 |
| DRD1 | SNF2-related DNA and ATP bd Helicase | Putative chromatin remodeling | Chromatin | 92 |
| CMT3 | Cytosine DNA methyl transf. Chromodomain Bromo-adjacent domain | Cytosine DNA methyl transferase | Chromatin | 88 |
| DRM1/2 | Cytosine DNA methyl transf. | Cytosine DNA methyl transferase | Chromatin | 88 |
| MET1 | Cytosine DNA methyl transf. Bromo-adjacent domain | Cytosine DNA methyl transferase | Chromatin | 88 |
| KYP | SET domain | H3K9 methyl transferase | Chromatin | 87 |
| | Zn^{II}-bd pre-SET domain | | | |
| | Post-SET domain | | | |
| | YDG domain | | | |
| | EF-hand | | | |
| SUVH2 | SET domain | H3K9 methyl transferase | Chromatin | 103 |
| | Zn^{II}-bd pre-SET domain | | | |
| | YDG domain | | | |

### Disease resistance in plants and animals

There is extensive evidence that the plant RNAi pathway plays essential roles in antiviral defense¹⁰⁴. Double-stranded RNA derived from viral genomes is diced into siRNAs by the redundant activities of both DCL4 (the major antiviral Dicer) and DCL2 (a surrogate of DCL4)¹⁰⁵. These siRNAs then incorporate into RISC (the RNA Induced Silencing Complex) to mediate slicing of viral transcripts and thereby reduce the overall viral load in plant cells¹⁰⁵. AGO1 is a likely effector protein of the siRNA loaded RISC, although other AGO paralogs might also be involved¹⁰⁶. A cell-to-cell and long distance signal for RNA silencing also accounts for the systemic spread of the antiviral innate immune response throughout plants¹⁰⁴. As a counter-defensive strategy, viruses encode suppressor proteins that are targeted against key processors and effectors of antiviral silencing. For instance, the P19 protein of tombusviruses sequesters siRNAs and prevents their use by RISC ¹⁰⁷, the 2b protein of *Cucumber mosaic virus* physically interacts with AGO1 and inhibits its cleavage activity ¹⁰⁶, and the P38 protein of *Turnip crinckle virus* strongly inhibits DCL4 activity¹⁰⁵. DCL3 (producing heterochromatic siRNAs) and DCL1 (producing miRNAs) do not appear to have a significant impact on plant virus accumulation.

Apart from antiviral defense, there is currently scant information available on the role of small RNA pathways in defense against other types of pathogens including bacteria and fungi, which account for major yield losses worldwide. In plants, fungal and bacterial resistance has been most thoroughly studied in the context of race-specific interactions, in which a specific resistance (R) protein protects the plant against a particular pathogen's race¹⁰⁸. This highly specific recognition leads to activation of defense responses and local cell death referred to as 'hypersensitive response' (HR). A well-characterized example of HR elicitation through race-specific interaction is provided by the *Arabidopsis RPS2* gene that confers resistance to *Pseudomonas syringae* pv. *tomato* strain DC3000 *(Pst* DC3000) producing the corresponding *AvrRpt2* elicitor protein. The presence of both RPS2 and AvrRpt2 components leads to resistance, whereas the absence of either component leads to disease¹⁰⁸.

Beside the race-specific interaction is a basal defense mechanism that plays a pivotal role in "non-host resistance", which accounts for the fact that most plants and animals are resistant to most pathogens. Basal defense relies on both constitutive and inducible responses. The inducible basal defense response is triggered upon perception of general elicitors known as 'pathogen-associated molecular patterns' (PAMPs). One such PAMP is a conserved 22 amino acid motif (flg-22) of the bacterial flagellin¹⁰⁹, which is recognized in several plant species, including *A*. *thaliana.* Perception of flg-22 triggers an innate immune response in plants that elevates resistance to the virulent *Pst* DC3000 strain¹¹⁰. As a counter-defensive strategy, bacterial pathogens have evolved to suppress basal defense responses by injecting TTS- proteins, refereed to as 'effectors'¹¹¹. These bacterial effectors are, therefore, virulence factors: their lack causes a loss of disease symptoms and a general inability of the pathogen to proliferate on leaves.

### miRNAs and the basal defense response

We have recently shown that miR393, a plant canonical and conserved miRNA, is rapidly induced by flg-22, leading to the repression of the entire signaling cascade that normally orchestrates the response to the phytohormone auxin¹¹². This report is Navarro, L., et al., Science (2006) 312:436-439, incorporated herein by reference. The resulting repression of auxin-signaling restricts bacterial growth, implicating auxin in disease susceptibility, and miRNA-mediated suppression of auxin-signaling in disease resistance. We hypothesized that miR393 was not an isolated example and that a large set of miRNAs may act as positive regulators of the plant defense response to pathogens.

### DISCLOSURE OF THE INVENTION

A specific spectrum of plant and animal miRNAs confer enhanced resistance to virulent pathogens. We show that plants deficient in miRNA accumulation are hyper-susceptible to non-virulent bacterial pathogens. As a corollary, we show that virulent bacteria have evolved strategies to suppress the miRNA pathway in plants, for example, by using some of the injected type-III secreted (TTS) proteins.

In one aspect, the invention is directed to a method for modulating the miRNA pathway in plants and animals which comprises introduction into a plant or animal a nucleic acid construct comprising a constitutive or pathogen responsive promoter operatively linked to a pathogen associated molecular pattern (PAMP)-responsive pri-miRNA sequence or a sequence which encodes a component involved in miRNA biogenesis or activity, optionally further including cis-regulatory elements within or 5' to said promoter. In one particular embodiment, the miRNA pathway modulated is other than that of miR393. In still another embodiment, said pathway regulated includes miR393.

In one embodiment, such modulation of the miRNA pathway in plants and animals is performed in such a way that adverse effects on plant and animal development are avoided or minimized.

In another embodiment, compositions and methods are provided for conferring on plants and animals enhanced pathogen resistance by selective modulation of the miRNA pathway. In one embodiment, the miRNA pathway modulated is other than miR393; in still another embodiment, the pathway includes that of miR393.

The invention provides methods to identifying compounds useful in the selective modulation of the miRNA pathway in plants and animals by using bacterial-derived suppressors of RNA-silencing as molecular probes. Such RNA-silencing factors likely interact directly with some of the bacterial silencing suppressors as described below.

The invention is also directed for identifying compounds or establishing genetic approaches to counteract bacterial suppression of RNA-silencing in both plant and animal cells. The overall approaches confer resistance to plant and animal pathogens.

Biochemical and genetic approaches known by those skilled in the art are used to identify additional Bss targets. These approaches include, but are not restricted to, yeast two-hybrid screen of plant cDNA libraries or biochemical pull downs using Bss tagged versions. The identified components likely interact (either physically or genetically) with, *e.g.,* DCL1, AGO1, HEN1, SERRATE and may be further used to manipulate specifically miRNA activities, using the methods described above. Importantly, these Bss proteins are also used to inhibit miRNA function in animal cells as observed with some viral suppressor of RNA-silencing derived from plant pathogenic viruses that are also functional in animal cells.

Another aspect of the invention takes advantage of the ability of Bss to suppress the silencing of transgenes thereby enhancing the production of recombinant proteins using hosts in which the Bss proteins are effective. In one embodiment, such hosts are plants. The recombinant host cell or plant is modified to contain an expression system for a desired protein, such as a therapeutic, fused to a Bss protein. Standard biochemical and molecular biology techniques are employed to construct suitable expression systems and to modify host cells for the production of a desired protein. Alternatively, separate constructs for the Bss protein and the desired protein may be used and co-transformed into the same cell or organism. By virtue of the presence of the Bss protein, cellular mechanisms that would silence its expression are inhibited. Thus, the level of production is enhanced and if the desired protein is produced with a tag sequence, purification is simplified.

Another application employs the availability of Bss proteins to identify compounds that repress bacterial infection by screening candidate compounds for ameliorating the effects of such proteins. The identified compounds may also be useful in other applications when silencing mechanisms are desirably enhanced. In this method, compounds that experimentally counteract Bss triggered suppression of RNA silencing and restore a normal vein chlorotic phenotype are selected. Endogenous compounds which have this effect may also be identified by mutagenizing plants in which silencing has been suppressed using Bss proteins and identifying genetic changes in plants where RNA silencing has been restored.

Furthermore, by constitutively or conditionally enhancing the activity of the components of the miRNA pathway identified as described herein above, increased resistance to a broad spectrum of pathogens is achieved in a variety of crop species. This method also allows inducible, enhanced resistance, which is desirable because it is not, or is less, detrimental to plant development and physiology in non-infected conditions.

### BRIEF DESCRIPTION OF THE DRAWINGS

Figures 1A-1D show the post-transcriptional RNA silencing pathways in plants.
   Figure 1A shows IR-PTGS pathway; Figure 1B shows S-PTGS pathway; Figure 1C shows micro (mi)RNA pathway; and Figure 1D shows trans-acting (ta)siRNA pathway; and Figure 1D shows trans-acting (ta)siRNA pathway.
Figure 2 shows transitive RNA silencing.
Figures 3A and 3B show chromatin-targeted RNA silencing, showing two of many non-mutually exclusive scenarios that possibly account for siRNA-directed chromatin modifications at endogenous loci. Both scenarios are based on circular and amplified schemes in which siRNA production and chromatin modification reinforce one another.
Figure 4 shows over-representation of W-box elements in the AtmiR393b promoter.
Figure 5 shows typical stem loop formations associated with pri-miRNA.
Figures 6A and 6B demonstrate the susceptibility of miRNA deficient mutants to fungal and bacterial pathogens. Figure 6A shows susceptibility to fungal pathogens and Figure 6B to bacterial pathogens.
Figures 7A-7E show experimental results that confirm that the miRNA pathway confers basal as well as non-host resistance to bacteria.
Figures 8A-8D demonstrate that infection by TuMV suppresses basal as well as non-host resistance to bacteria.
Figures 9A-9C demonstrate that the virulent strain Pst DC3000 can suppress transcriptional induction of some PAMP-responsive miRNAs.
Figures 10A-10D provide experimental results demonstrating that Pst DC3000 encodes Type III secreted protein that suppress miRNA biosynthesis and/or activity.
Figures 11A-11F provide experimental results demonstrating that HopT1-1 suppresses a silencing function mediated by RISC^{miRNA}_{.}
Figures 12A-12D present experimental results demonstrating that overexpression of HopY1 suppresses RISC^{miRNA} function.
Figures 13A-13E present experimental results showing that HopU1 suppresses miRNA and siRNA triggered translational inhibition.

### MODES OF CARRYING OUT THE INVENTION

By exploring the elements of pathogen resistance and its relationship to silencing pathways, we have determined a number of factors and methods to enhance resistance of plants and animals to pathogen infection, to identify components critical to resistance, and to screen for compounds or agents that are helpful in enhancing resistance. For example, known components of the miRNA pathway (*e.g.,* DCL1, AGO1, HEN1, SERRATE) and the components identified as disclosed herein above (*e.g.,* AtGRP7, GRP8) are modified such that they become resistant to the action of Bss proteins. Engineering resistant alleles of these components is achieved according to methods known by those skilled in the art, including, but not limited to, site-directed mutagenesis of key amino acids and transgenesis, as well as Targeted Induced Local Lesions in Genomes (TILLING) of non-transgenic crop species. The method disclosed allows for natural and/or engineered resistance to Bss action and thereby confers enhanced basal defense to crop species against virulent pathogens.

The sequences identified in Example 1 below and listed in Table 2 that express pri-miRNA are operatively linked to suitable promoters and used to modify plants to confer resistance to virulent infection. One of these sequences, miR393 was earlier shown by us to be transcriptionally induced by flg-22. Its constitutive over-expression confers enhanced resistance to virulent *Pst* DC3000 (Navarro, L., et al., Science (2006) 312:436-439. Additional pri-miRNA sequences thus identified are used to elevate the plant resistance to a broad spectrum of pathogens. Individual or groups of these sequences, designated patho-miRNAs, are expressed transgenically in plants using methods known by those skilled in the art, such as for the overexpression of one of the two miR393 *loci* Navarro, L., *et al.* (*supra*)*.* Expression of these sequences (+ 40 nt upstream and downstream) is either constitutive or, preferably, is driven by promoters that are known to be broadly responsive to bacterial, fungal and viral pathogens. Examples of such promoters include, but are not restricted to, WRKY6 and PR1. The method allows inducible, enhanced resistance, which is desirable because it is not, or is less, detrimental to plant development and physiology in non-infected conditions.

Moreover, several of these patho-miRNAs are conserved across plant species (monocots and dicots), indicating that *Arabidopsis-*derived patho-miRs will be directly effective in a large variety of crops.

Constructs are prepared wherein, in one embodiment, a constitutive or pathogen responsive promoter (including but not limited to, for example, the WRKY6 promoter, the PR1 promoter and the like) is operatively linked to a nucleic acid sequence which is transcribed into one or more individual patho-miR sequences to confer enhanced resistance to unrelated pathogens in various plant species, including crops.

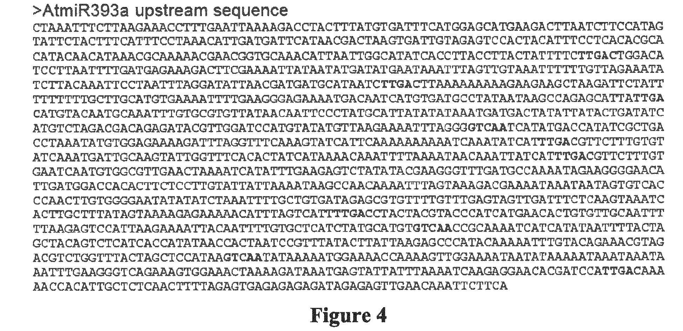

Computational analyses reveal an over-representation of specific regulatory elements located within 1.5 or 2 kilobases (kb) upstream of the patho-miR stem-loop structures. Some of those regulatory elements have been previously described: as an example, the flg-22-responsive *At-miR393a* promoter contains 10 W-box elements, whereas the *At-miR393b* promoter, that is not responsive to flg-22 treatment, contains only 3 W-box elements. See Figure 4. Figure 4 shows over-representation of the W-box element within At-miR393a promoter sequence. 1.5 Kilobase upstream of the *At-miR393a* stem loop structure was extracted and analyzed for the representation of W-box elements. In red in the figure and underlined in the sequence provided below is the core sequence of the W-box element. We also identified an over-representation of the characterized auxin-responsive element AuRE as well as the RY element within subsets of patho-miR promoters. In addition, we found an over-representation of novel elements referred to as Flg-22-Induced MiRNA (FIM) element. These latter represent an original source of pathogen-responsive DNA elements to be used and manipulated in conventional approaches to enhanced pathogen resistance. Such approaches include the generation of chimeric DNA segments containing multiple copies of these known or novel cis-regulatory elements. The method hereby disclosed is thus used to specifically hyper-induce the expression of protein-coding genes or non-protein-coding genes (*e.g.,* miRNA genes) upon pathogen infections, in plants, including crops.

Thus, an artificial pathogen-responsive promoter carrying multiple copies of FIM elements, or alternatively a native patho-miR promoter, operatively linked to a coding sequence from plant defense genes or non-coding sequences (*e.g.,* miRNA sequences) that play a role in disease resistance may be used to control expression for disease resistance. This approach allows the conditional and effective expression of protein-coding genes or non-protein coding genes during pathogen infections.

Because the miRNA pathway is the prominent RNA silencing pathway in higher vertebrates, and because mammalian cells are commonly infected by TTS-bacterial pathogens, similar mechanisms of defense/counter-defense are operational in mammals. Recent findings also highlight the key role of miRNAs in the animal innate immune response. By using miRNA sensor genes akin to those exemplified herein for *Arabidopsis,* but incorporating components of human or other animal pathogenic bacteria *(e.g., Yersinia pestis, Pseudomonas aeruginosa, Shigella flexneri)* methods and compositions that interfere with miRNA activity/biogenesis in human or animal cultured cells are tested. Individual TTS-effector proteins that account for this interference and are key virulence factors that negate the mammalian innate immune responses to life-threatening pathogens are thus identified. This technology provides a method whereby transfection of plasmids encoding individual TTS-proteins (together with their corresponding chaperone), in human cells expressing an miRNA sensor which comprises control sequences for expression of animal generators of miRNA operatively linked to a reporter sequence construct permits identification of therapeutic agents. Molecules that interfere with TTS-protein activity without damaging cellular miRNA functions are selected as candidates for further study and drug development. Cultured human cells co-expressing the miRNA sensor and bacterial TTS-proteins are subjected to high-throughput delivery of a large collection of active molecules. Those molecules suppressing miRNA sensor expression are isolated and further tested for their potential therapeutic effect as described above.

Bss proteins from mammalian pathogens identified through the methods described hereinabove are also useful proteins to isolate animal components involved in miRNA biogenesis and/or activity using biochemical and genetic approaches known by those skilled in the art. These approaches include, but are not restricted to, yeast two-hybrid screen of mammalian cDNA libraries, biochemical pull downs and forward genetic screens for loss of Bss function in Ethyl-methyl sulfonate (EMS)-mutagenized cells. The identified components likely interact (either physically or genetically) with, *e.g.,* mammalian Dicer, mammalian Drosha, mammalian TBP and may be further used to manipulate specifically miRNA activities in mammals.

Using the high-throughput screening methods described herein above, active molecules are identified that promote or enhance the activity of known components of the mammalian miRNA pathway (*e.g.,* mammalian Dicer, mammalian Drosha, mammalian TBP) and of the components of the mammalian miRNA pathway identified herein above. Treatments of human or other animal cells with such molecules enhance the basal defense to a broad range of pathogens.

By constitutively or conditionally enhancing the activity of the components of the mammalian miRNA pathway identified herein above, increased resistance to a broad spectrum of pathogens is achieved in a variety of host mammalian cells. Conditional expression is conferred by, for example, the NFκB promoter. The method hereby disclosed thus allows inducible, enhanced resistance, which is desirable because it is not, or is less, detrimental to development and physiology in non-infected conditions. The methods of this invention are applicable to all infections involving injection/delivery of factors of parasitic origin. Examples include biotrophic fungi such as *E. cichoracearum,* a Powdery mildew that over-accumulates on both *hen1-1* and *dcl1-9* mutants, and therefore likely secrete effector proteins to suppress RNA-silencing pathways.

The Bss proteins can also be used to inhibit miRNA function so as to alter physiological and developmental processes normally orchestrated by these small RNA molecules. This is achieved by fusing Bss coding sequences to tissue-specific plant promoters known by those skilled in the art (driving expression in, *e.g.,* roots, leaves, stem, inflorescences), which may also include the patho-miR promoter elements identified herein. The method hereby disclosed thus allows tissue-specific expression of Bss proteins for the spatial and temporal control of miRNA function.

Bss proteins are thus used to suppress transgene silencing and allow high expression of any proteins of interest (*e.g.,* therapeutic molecules). In one application, single or multiple constructs carrying the gene(s) of interest and a construct carrying a Bss cDNA (*e.g.,* HopU1) fused to strong promoter are co-delivered *in planta* (using Agrobacterium-mediated transient assay). This transient assay can be performed in leaves from *N. benthamiana,* in which transient assay is more efficient, or preferentially in plant cell cultures (such as *Arabidopsis* Col-0 cells). The protein(s) of interest are further purified using methods known by those skilled in the art and their biological activity tested. The use of plant cell cultures will allow the production of large amounts of recombinant proteins. Furthermore, this system will facilitate the protein purification step as the starting materials will contain reduced amounts of plant-derived components that are unwanted in protein purification (*e.g.,* polyphenols).

HopN1 mutated versions (with C172S, H283A or D299A substitutions, abolished in their cysteine protease activity) will be particularly useful for this approach as these mutant versions will not be, or less be, recognized by resistant proteins (which trigger host programmed-cell death that could impact negatively on recombinant protein yields).

Another application is in a method for identifying compounds that counteract bacterial suppression of RNA-silencing. Bss proteins and a miRNA sensor construct (*e.g.,* 171 sensor) or the SUC-SUL transgene (or any other silencing reporter constructs) are co-expressed stably in plants and sprayed with a library of compounds to identify molecules that counteract Bss-triggered suppression of RNA-silencing. As an example, the SUC-SUL plants expressing HopT1-1 display attenuated vein chlorotic phenotype due to the suppression of AGO1-containing RISC activity. These plants are sprayed with a battery of molecules to identify compounds that counteract Bss-triggered suppression of RNA-silencing and likewise restore a normal vein chlorotic phenotype. Such molecules are used to confer broad spectrum resistance to pathogens in various plant species including crops.

Alternatively, plants are mutagenized (using methods known by those skilled in the art such as EMS mutagenesis) to identify mutants in which RNA-silencing is restored. For example, SUC-SUL plants that express HopT1-1 are mutagenized and mutants that restore a normal vein chlorotic phenotype isolated. The corresponding genes are further identified using methods known by those skilled in the art (*e.g.,* map-based cloning). Orthologs of such genes, in various plant species including crops, are identified and methods used to knock-out or knock-down these particular genes applied.

Because animal pathogenic bacteria also use the type-three secretion system to deliver key virulence proteins, animal pathogenic bacteria (including human pathogenic bacteria) have also evolved to suppress RNA-silencing. Such bacterial virulence genes are fused to a strong promoter (*e.g.,* CMV) and delivered to animal cells using known methods. Levels of endogenous miRNAs as well as miRNA targets are monitored to identify proteins that suppress RNA-silencing (as performed in the plant experimental systems described above). These Bss proteins, derived from animal pathogenic bacteria, are further co-expressed in animal cells with an miRNA sensor construct. Molecules that -restore a normal expression of the miRNA sensor are further identified and used to confer enhanced resistance to bacterial pathogens. Such molecules potentially represent substitutes for antibiotics. Alternatively, the above animal cells are mutagenized using methods known by those skilled in this art and the corresponding genes identified. Methods that allow knock-down or knock-out of such genes are used to elevate resistance to bacterial pathogens. Such methods include but are not restricted to, the use of artificial siRNAs directed against endogenous repressors of Bss-triggered suppression of RNA-silencing.

The following are non-limiting aspects of the invention.
1. A method for modulating the miRNA pathway in plants and animals which comprises introduction into a plant or animal a nucleic acid construct comprising a constitutive or pathogen responsive promoter operatively linked to a miRNA encoding sequence or a sequence which encodes a miRNA effector protein, optionally further including cis-regulatory elements within or 5' to said promoter.
   This method may be designed so that adverse effects on plant and animal development are avoided or minimized, and may provide enhanced pathogen resistance upon expression in said plant or animal, for example, by employing inducible or conditional control sequences.
   In this method, the effector protein is a protein set forth in Table 1, *e.g.,* DCL1, AGO1, SERRATE, and/or HEN1.
2. A method for identifying compounds useful in the selective modulation of the miRNA pathway in plants and animals by monitoring expression in a plant or animal cell of a sensor transgene reporting the activity of endogenous miRNAs in response to perturbation caused by exposure of said plant or animal cell to a candidate compound.
3. The invention also provides a bacterial silencing suppressor, *e.g.,* HopN1, HopH1, HopPto, HopT1, HopY1, or HopU1 or a functional derivative thereof.
   These may be used in a method for selectively modulating miRNA expression in a cell by contacting said cell with the bacterial silencing suppressor.
4. A method for identifying miRNAs associated with a plant or animal response to a pathogenic elicitor. A plant or animal or plant or animal cell is exposed to a pathogenic elicitor and compared with a substantially identical plant or animal or plant or animal cell not exposed to the pathogenic elicitor. Transcriptional fluctuation of computationally predicted or experimentally validated miRNAs as primary transcripts is monitored and compared. The invention is also directed to an isolated pathogen-elicited miRNA so identified.
   The invention is also directed to a method for conferring enhanced pathogen resistance on a plant or animal or plant or animal cell by effecting expression of a pathogen-elicited miRNA identified as above. The expression may be under the operative control of a promoter which is activated on exposure to a pathogen, *e.g.,* the WRKY6 promoter or the PR1 promoter.
   One or more cis-acting regulatory elements may also be provided upstream of or incorporated within said promoter and miRNA encoding sequence. The cis-acting regulatory element may be a W box, an AuxRE element, an RY element, an FIM element, multiples of these elements or combinations thereof.
5. A method for determining the role of a miRNA by suppressing miRNA in a cell that otherwise exhibits expression of said miRNA by contacting said cell with a bacterial suppressor of silencing (Bss).
6. A method for modulating the resistance in an animal to infection by a pathogen which comprises either enhancing the expression of miRNA pathway components suppressed by proteins secreted by said pathogen or increasing the resistance of miRNA pathway components to suppression by proteins secreted by said pathogen.
7. A method of conferring resistance in a plant to pathogens which comprises selecting plants with miRNA pathway components resistant to the action of suppressors of silencing, such as a bacterial suppressor of silencing, and plants selected by this method.
8. A method for specifically manipulating miRNA accumulation in order to alter physiological and developmental processes normally orchestrated by those molecules by fusing a Bss coding sequence to a tissue-specific plant promoter driving expression in roots, leaves, stem, inflorescences, optionally including patho-miR promoter elements to achieve spatial and temporal control of miRNA activity.
9. A method for exploiting Bss triggered suppression of RNA-silencing activity without recognition by the plants. This includes generating mutants that can still suppress the miRNA and siRNA pathways but can no longer be perceived by plant derived R genes. HopN1 mutated in the catalytic triads can be used because these mutants suppress miRNA biogenesis at the same level as does HopN1 wildtype but are not perceived by R proteins.
10. A method to identify components of the siRNA and/or miRNA pathway by using Bss proteins as molecular probes in both plant and animal cells and retrieving components that physically interact with such proteins.
11. A method to suppress miRNA function in animal cells by using Bss proteins derived from plant pathogenic bacteria such as Pst DC3000. The Bss proteins are fused to constitutive human promoters and transfected in animal cells in order to suppress miRNA function. This may be used in combination with recombinant expression of desired proteins.
12. A method for overexpressing recombinant proteins (*e.g.,* human proteins with therapeutic activity) in plant cells. This will be performed by expressing Bss proteins that inhibit transgene silencing. This approach is preferentially performed in cell cultures using an Agrobacterium transient assay. The purified recombinant proteins are further tested for their biological activities and used as medicines.
13. A method for identifying molecules that promote transcription of endogenous factors involved in miRNA biogenesis of activity. One method employs constructs in which control sequences of miRNA expression are coupled to reporter sequences. Another method employs reporter constructs that are silenced by miRNA. In the first case, molecules or genetic alterations that increase expression of reporters are enhancers of miRNA expression. In the second case, the opposite is true.
14. A method for identifying compounds that counteract Bss-triggered suppression of RNA-silencing in either plant or animal cells. This approach comprises the use of plant or animal cells that co-express an RNA-silencing reporter construct with a Bss protein. Molecules that restore normal expression of the RNA-silencing reporter gene are identified and used to elevate resistance to bacteria in plants and animals. Such molecules may be used as antibiotics in animal cells. Alternatively, endogenous repressors of Bss-triggered suppression of RNA-silencing are identified by using mutagenesis of the said plant or animal cell lines co-expressing a RNA-silencing reporter construct and a Bss protein.
15. The approaches described herein are used to identify secreted proteins from parasites that could suppress RNA-silencing pathways.

The following examples are offered to illustrate but not to limit the invention.

### EXAMPLES

The Examples show that 1) a specific spectrum of plant miRNAs confers enhanced resistance to virulent pathogens; and that 2) plants deficient in miRNA accumulation are hyper-susceptible to virulent and non-virulent bacterial pathogens and that, as a corollary; virulent bacteria must therefore have evolved strategies to suppress the miRNA pathway, for instance by using some of the injected type-III secreted (TTS) proteins.

All the results below were generated in the model species *Arabidopsis thaliana,* as illustrative of plants in general including crops. While the specifics of the examples that follow are provided to fully enable those skilled in the art to understand and practice this invention, to provide the best mode for practicing this invention, and to supply a thorough written description of the invention, the invention should not be construed as being limited to the specifics as outlined in these examples.

### Example 1

### A specific spectrum of plant miRNAs is up-regulated by flg-22 peptide

A set of primary miRNA transcripts was identified using total RNA fractions isolated from naive or flg-22-treated plants. flg-22 Is a peptide eliciting a response to pathogen-associated molecular patterns (PAMP). Reverse complements of 60nt long sequences located upstream of predicted and validated pre-miRNA stem loops were spotted onto an array and used as probes to profile primary miRNA transcripts upon flg-22 treatment.

As noted in the Background above, miRNA is generated initially from primary RNA (pri-miRNA) transcripts which are subsequently cleaved to pre-miRNA transcripts from which miRNA transcripts are formed. Because the pri-miRNA transcripts are characterized by a stem loop structure, as shown in Figure 5, identification of the stem loops in predicted RNA structures permitted identification of putative locations for pri-miRNA locations in the genome.

The method described above was applied to wildtype plants as well as to various silencing mutants, including *dcl4-1* and *dcl1-9.*

We identified 68 pri-miRNAs, that are significantly up-regulated upon flg-22 treatment in at least one genetic background (either Col-0, *dcl1-9* or *dcl4-1* backgrounds). Some of these primary miRNA transcripts have not yet been described in other reports indicating that many miRNAs are specifically expressed upon biotic stress treatments but not in standard growth conditions. Most of these precursors give rise to extensive fold-back structures.

The sequences of the 68 flg-22-induced miRNA precursors are shown in Table 2. The sequences highlighted in bold are the predicted mature miRNA sequences.

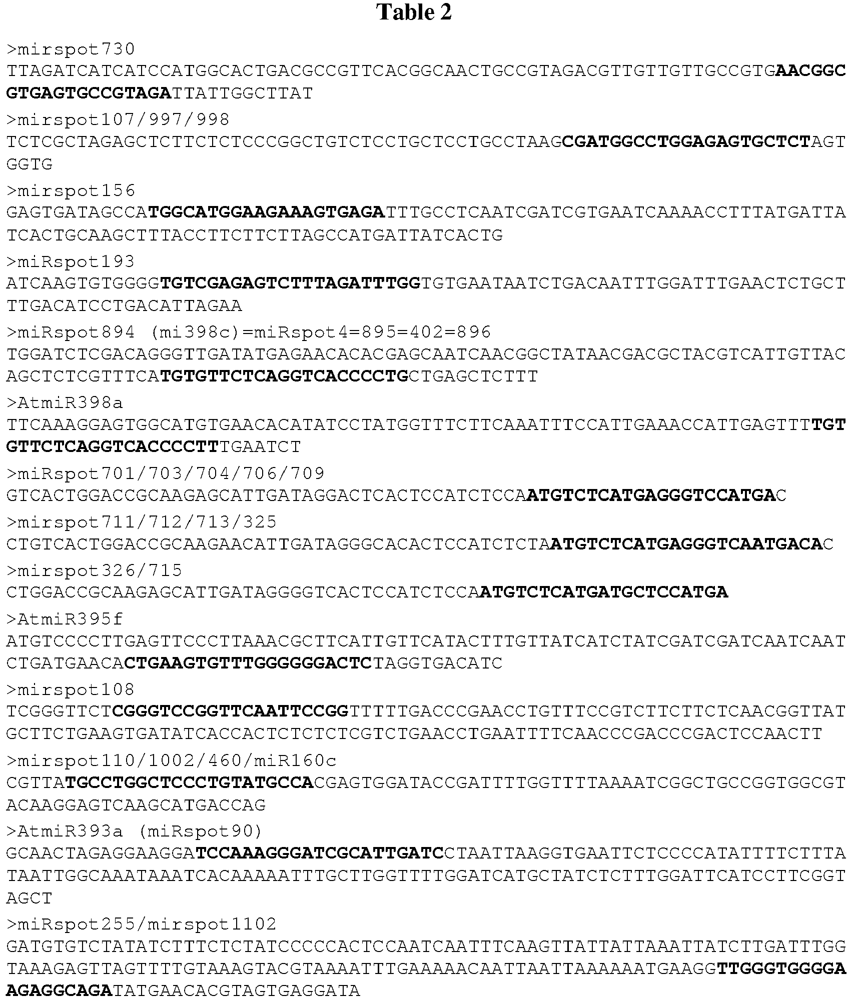

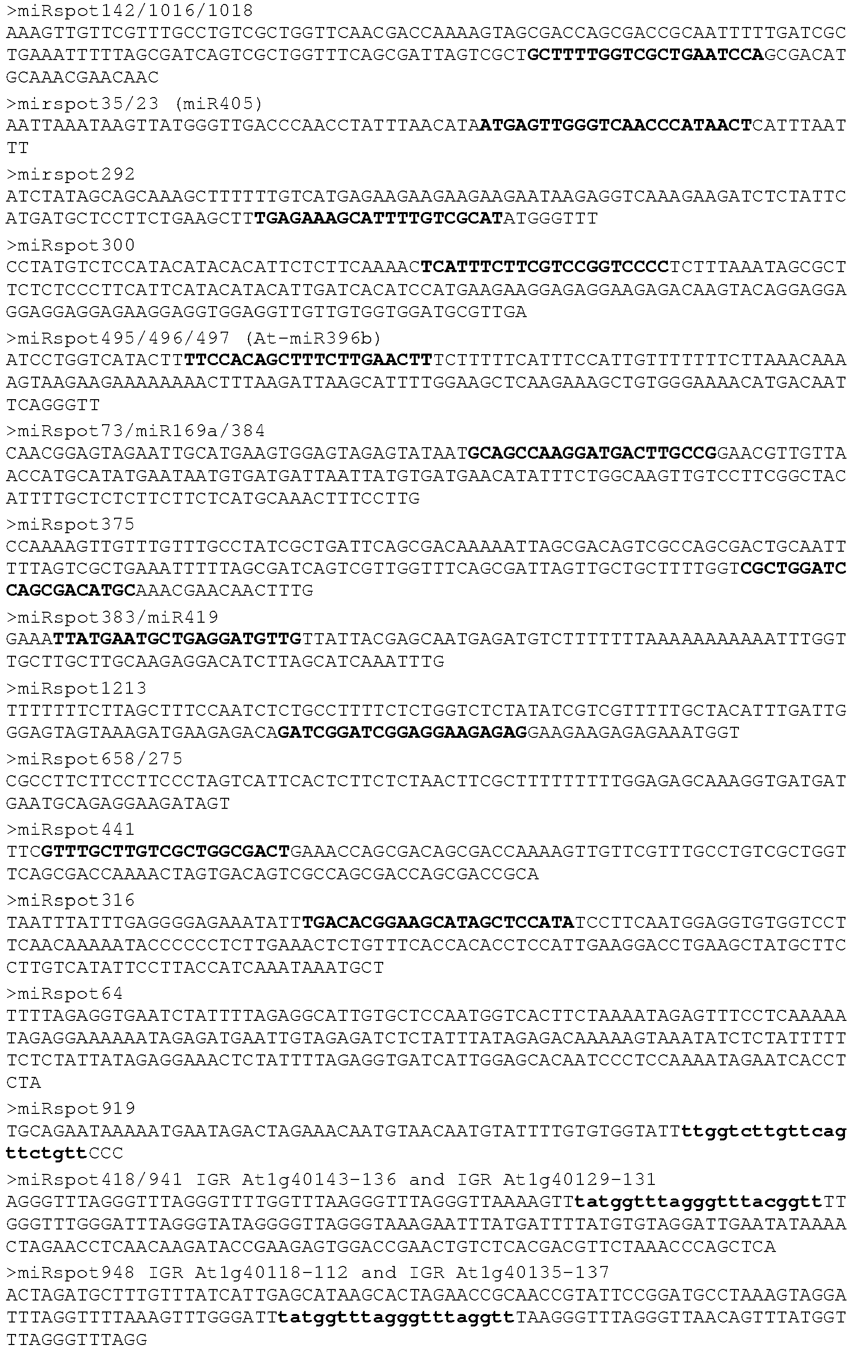

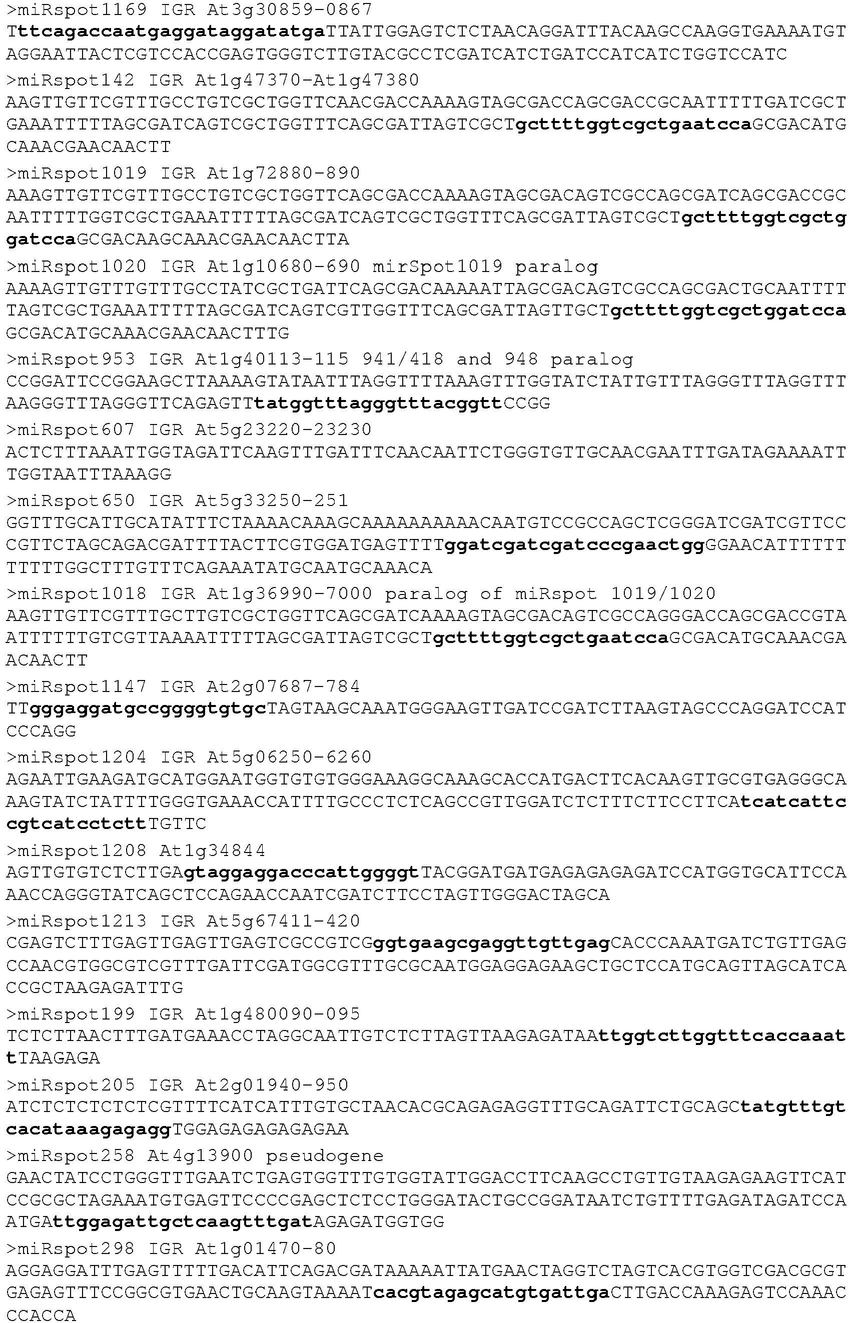

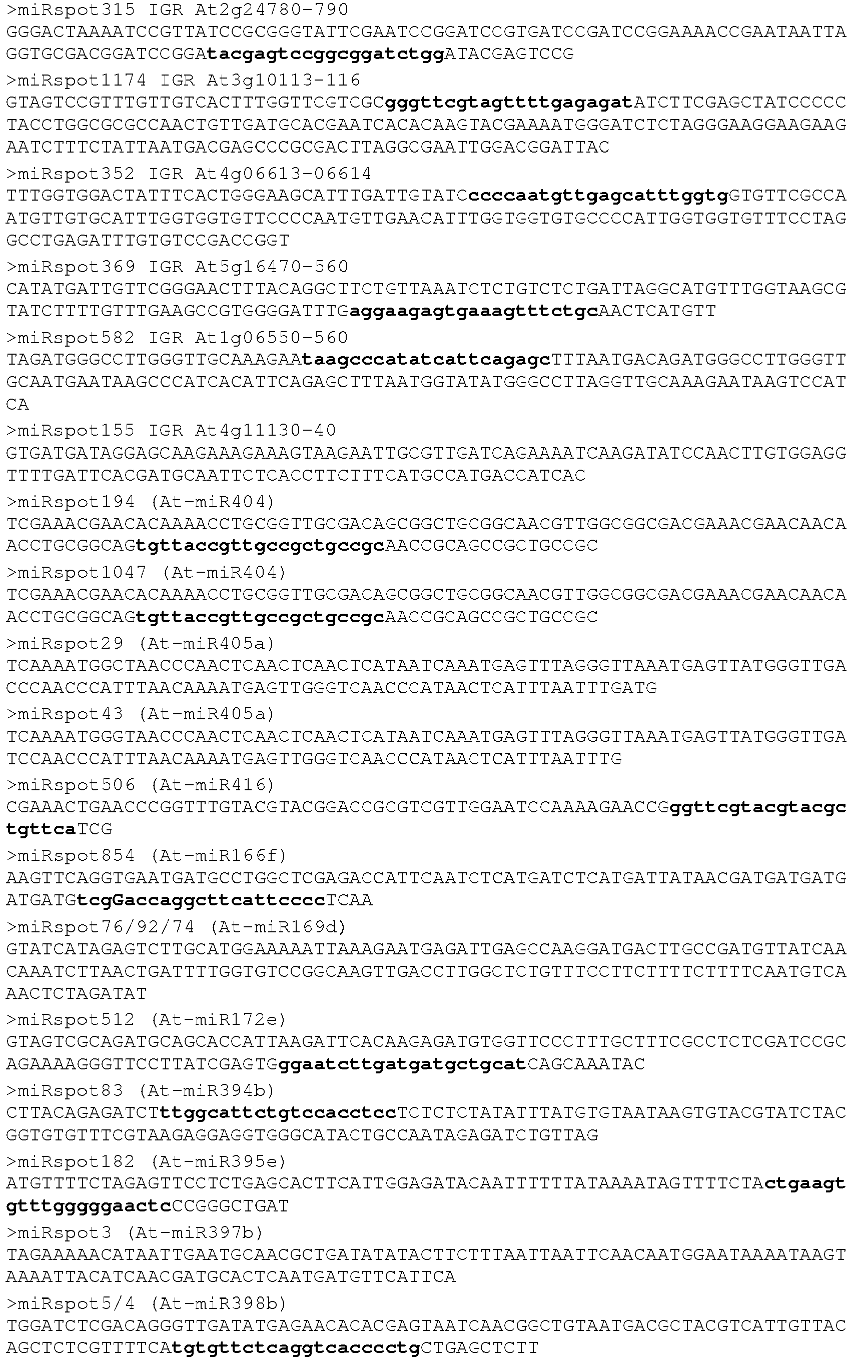

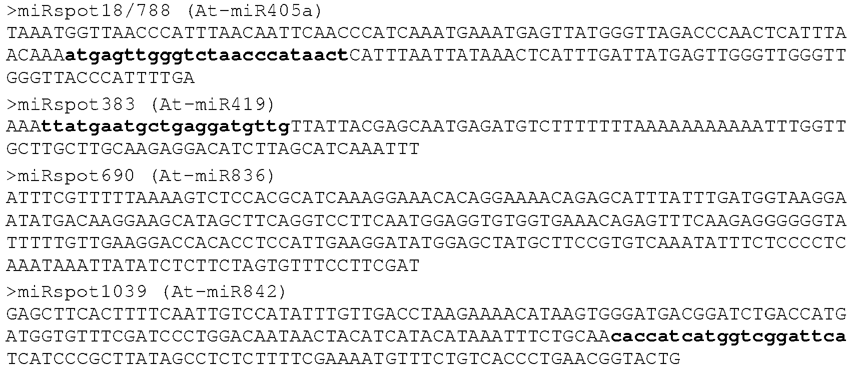

Among the flg-22-induced pri-miRNAs, many corresponding mature miRNA sequences are conserved in maize and rice. The afore-mentioned method can also be used to identify the full spectrum of miRNAs elicited by general elicitors exhibiting pathogen-associated molecular patterns (PAMPs) such as flg-22.

miRNA primary transcripts suppressed by suppressor proteins such as those associated with virulent bacteria can be identified by challenging wildtype Col-0 leaves with Pst DC3000 *hrcC* (a Pst DC3000 bacterium that cannot inject suppressor proteins into the host cells) and virulent Pst DC3000 (which can) for 6 hours and by further selecting miRNA primary transcripts (using the above method) that are up-regulated by Pst DC3000 *hrcC* but not by virulent Pst DC3000 as described below. Because these pri-miRNAs are transcriptionally repressed by Pst DC3000 TTS proteins, they likely act as key components of the antibacterial defense response. The afore-mentioned method can also be used to identify the full spectrum of miRNA transcripts that are up-regulated by unrelated biotrophic and necrotrophic pathogens as well as abiotic stresses.

### Example 2

### Plant mutants with compromised miRNA accumulation are more susceptible to virulent pathogens

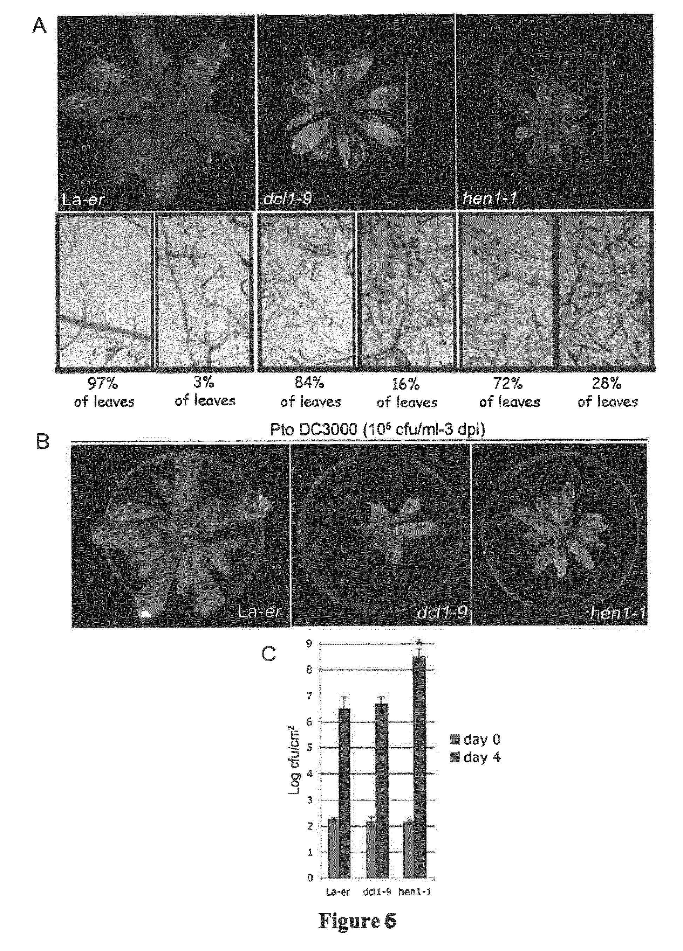

We demonstrated the involvement of the miRNA pathway in plant disease resistance, by challenging *Arabidopsis hen1-1* and *dcl1-9* mutants with a virulent Powdery mildew *Erysiphe cichoracearum* or with virulent *Pst* DC3000. We found that *hen1-1* is hyper-susceptible to both the fungus and the bacterium, and that *dcl1-9* displays enhanced susceptibility to *Erysiphe cichoracearum* and enhanced disease symptoms upon virulent *Pst* DC3000 infection (Figure 6A, B, C). In Figure 6A, both *hen1-1* and *dcl1-9* are hyper-susceptible to *Powdery mildew.* Six week-old La-er, *hen1-1* and *dcl1-9* plants were infected with *E. cichoracearum* (UEA isolate) spores and fungal growth assessed visually 10 dpi (upper panel). Fungal growth and sporulation were also assessed microscopically after leaf staining with trypan blue at 2-3 dpi (bottom panels).

In Figure 6B, both *hen1-1* and *dcl1-9* display enhanced disease symptoms upon *Pst* DC3000 infection. La-*er*, *hen1-1* and *dcl1-9* were challenged with Pst DC3000 of 10⁵ colony forming units (cfu/ml) and bacterial disease symptoms assessed visually 3 dpi.

As shown in Figure 6C, *Hen1-1* displays higher *Pst* DC3000 titers. La-er, *hen1-1* and *dcl1-9* were treated as in Figure 6B and bacterial growth measured 4 dpi.

Constitutively or conditionally enhancing the activity/expression of components involved in miRNA biogenesis or activity, such as DCL1, AGO1, SERRATE, HEN1 using the methods above increases resistance to a broad spectrum of pathogens is achieved in a variety of plants, including crop species. The method disclosed thus allows inducible, enhanced resistance, which is desirable because it is not, or is less, detrimental to plant development and physiology in non-infected conditions.

*Arabidopsis* transgenic lines carrying 1.5 Kb upstream regions from genes involved in miRNA biogenesis and/or activity (*e.g.,* DCL1) are fused to a reporter sequence. These transgenic lines are used to screen for chemical compounds that trigger up-regulation of reporter mRNA or protein

This is achieved by monitoring mRNA levels (using methods known by those skilled in the art such as Northern analysis, semi-quantitative RT-PCR analysis or quantitative RT-PCR analysis) after exposure of these transgenic lines to a library of chemical agents. Molecules that induce reporter mRNA (or protein) levels are further used to confer antibacterial and antifungal resistance in a variety of plant species including crops.

### Example 3

### miRNA-Deficient Plants are Susceptible to Non-Virulent Bacteria

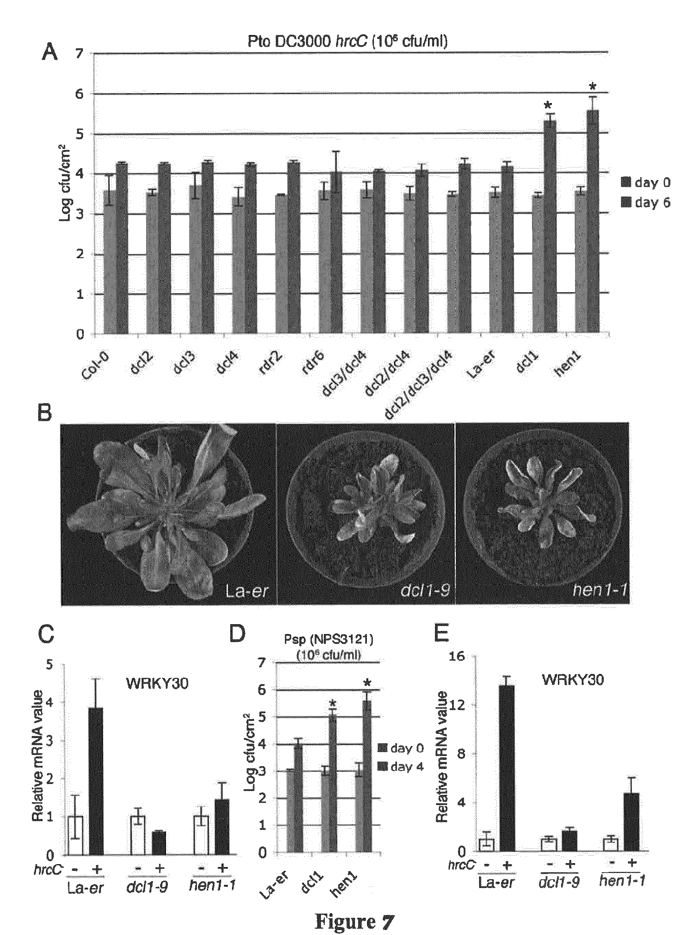

The induction, by flg-22, of a subset of patho-miRs (see Example 1) suggests that the miRNA pathway plays a pivotal role in basal resistance to pathogens. We challenged the *dcl1-9* and *hen1-1* mutants of *Arabidopsis* with *Pst* DC3000 deficient in type three secreted (TTS) protein. *Pst* DC3000 *hrcC,* a strain which in wildtype *Arabidopsis* is unable to mount an effective infection, was injected into these mutants. Both mutants exhibited full disease symptoms, resembling the phenotype induced by virulent bacteria on wildtype *Arabidopsis* (Figures 7A, 7B).

In Figure 7A, both *hen1-1* and *dcl1-9,* but not siRNA-deficient mutants, are compromised in basal resistance. Col-0, *dcl2-1, dcl3-1, dcl4-2, Jcl3-1*/*dcl4-2, dcl2-1ldcl4-1, dcl2-1ldcl3-1*/*dcl4-2, rdr6-1, rdr2-1, La-er, hen1-1* and *dcl1-9* were challenged with hrcC⁻ Pst DC3000 at 10⁶ colony forming units (cfu/ml) concentration and bacterial titers measured 6 dpi.

In Figure 7B, both *hen1-1* and *dcl1-9* displayed disease symptoms when inoculated with *hrcC Pst* DC3000 strain. Inoculations were performed as in Figure 7A. and bacterial disease symptoms were assessed visually.

We found that induction of WRKY30 (a well-characterized basal defense marker gene) was compromised in both *hen1-1* and *dcl1-9* mutants challenged with *Pst* DC3000 *HrcC⁻* (Figure 7C), indicating a loss of basal defense response in those plants. In Figure 7C, the induction of the WRKY30 gene was impaired in both *hen1-1* and *dcl1-9* treated with *hrcC Pst* DC3000 strain. Inoculations were performed as in Figure 7A and WRKY30 levels were analyzed by quantitative RT-PCR (qRT-PCR) on 12 hours post inoculated samples.

Likewise, both *henl-1* and *dcl1-9* sustained infection from the non-host bacterial pathogen *Pseudomonas syringae pv. phaseolicola* (NPS3121) (Figure 7D), which is normally virulent on beans, but not on *Arabidopsis.* In Figure 7D, both *hen1-1* and *dcl1-9* are compromised in non-host resistance. La-*er*, *hen1-1* and *dcl1-9* were challenged with *Pseudomonas syrinage* pv. *phaseolicola* (NPS3121) at 10⁶ colony forming units (cfu/ml) concentration and bacterial titers measured 4 dpi.

WRKY30 induction was also impaired on challenge with *Pseudomonas syringae pv. phaseolicola* (NPS3121) (Figure 7E). In Figure 7E, the induction of the WRKY30 gene was impaired in both *hen1-1* and *dcl1-9* treated with *Pseudomonas syrinage* pv. *phaseolicola* (NPS3121). Inoculations were performed as in Figure 7D and WRKY30 levels were analyzed by quantitative RT-PCR (qRT-PCR) on 12 hours post inoculated samples. Importantly, similar results were obtained with the non-pathogenic *Pseudomonas fluorescence* (data not shown).

We also found that TuMV virus (that encodes the viral suppressor of silencing P1-HcPro) suppresses basal resistance to non-virulent bacteria Pst DC3000 *hrcC* and *Pseudomonas syringae pv. phaseolicola* (NPS3121) suggesting that suppression of the RNA-silencing machinery might play a pivotal role in polymicrobial infection.

Figure 8A shows TuMV infection rescues bacterial disease symptoms of Pst DC3000 *hrcC* mutant. Wildtype Col-0 plants were treated with 10⁶ cfu/ml of Pst DC3000 *hrcC* for 6 days (left panel) or SAP inoculated with TuMV for 7 days and further treated with 10⁶ cfu/ml Pto DC3000 *hrcC* for another 6 days (right panel).

Figure 8B shows TuMV infection rescues bacterial growth of Pst DC3000 *hrcC* mutant. Wildtype Col-0 plants were treated as in Figure 8A and bacterial titers were measured 6 dpi.

Figure 8C shows TuMV infection rescues bacterial disease symptoms of the non-host *Pseudomonas syrinage* pv. *phaseolicola* (NPS3121). Wildtype Col-0 plants were treated with 10⁶ cfu/ml of *Pseudomonas syrinage* pv. *phaseolicola* (NPS3121) for 4 days (left panel) or SAP inoculated with TuMV for 7 days and further treated with 10⁶ cfu/ml *Pseudomonas syrinage* pv. *phaseolicola* (NPS3121) for another 4 days (right panel).

Figure 8D shows TuMV infection rescues bacterial growth of *Pseudomonas syrinage* pv. *phaseolicola* (NPS3121). Wildtype Col-0 plants were treated as in Figure 8C and bacterial titers were measured 4 dpi.

### Example 4

### Bacterial Silencing Proteins Suppress miRNA Identification of Bacterial Silencing Suppressors (Bss)

We also conclude from the results of Example 3 that pathogens, such as viruses and virulent bacteria, must have evolved strategies to suppress components of the silencing machinery.

To test this hypothesis we first investigated whether virulent Pst DC3000 could suppress the transcription of PAMP-responsive miRNAs. We measured the impact of Pto DC3000 effector (TTS protein) delivery on pri-miRNA expression. Virulent Pto DC3000, or its non-virulent counterpart Pto DC3000 *hrcC,* were inoculated onto *Arabidopsis* Col-0 plants and the levels of several pri-miRNAs induced by Pto DC3000 *hrcC* (referred to as PAMP-responsive) were then monitored over a 6 hour timecourse. In virulent Pto DC3000-treated plants, induction of the PAMP-responsive pri-miR393a/b and pri-miR396b was significantly suppressed at 6 hour post inoculation (hpi), as was the induction of the basal defense markers *WRKY30* and *Flagellin Receptor Kinase 1* (*FRK1*) used as internal controls (Figure 9A). Figure 9A shows induction of PAMP-responsive miRNAs is suppressed by virulent Pst DC3000. Wildtype Col-0 leaves were inoculated with Pst DC3000 *hrcC* at 2x10⁷ cfu/ml concentration and the levels of PAMP-responsive primary miRNA transcripts were monitored by semi-quantitative RT-PCR over a 6 hours timecourse. Pri-miR166a was used as a negative control in this experiment. By contrast, the levels of the PAMP-insensitive pri-miR166 and pri-miR173 remained unchanged.

We then used the previously described miR393a-p::eGFP and miR393b-p::eGFP transgenic lines, which report miR393 transcriptional activity. At 6 hpi, Pto DC3000 *hrcC* caused an increase in eGFP mRNA levels in both transgenic lines, indicating the presence of PAMP-responsive elements upstream of *MIR393a* and *MIR393b* (Figure 9B). Figure 9B shows Pst DC3000 suppresses miR393a/b induction at the transcriptional level. Transgenic lines expressing either miR393a-p::eGFP or miR393b-p::eGFP transgenes were treated as in Figure 9A for 6 hours and the levels of eGFP transcript were monitored by RT-qPCR. The receptor-like kinase FRK1 was used as a positive control in this experiment. However, this induction was compromised by Pto DC3000, as was the induction of the FRK1 control. Because the two bacteria differ in their ability to deliver TTS effectors into host cells, these results suggest that some injected bacterial proteins suppress specifically the transcriptional activation of pri-miR393a/b and perhaps of other PAMP-responsive miRNAs.

To identify such bacterial proteins, we delivered individual Pst DC3000 effectors (driven by the strong 35S promoter) in *efr1* mutant leaves, a mutant in which *Agrobacterium* transient assay is facilitated, and monitored PAMP-responsive pri-miRNA levels. Delivery of AvrPtoB significantly reduces pri-miR393a/b and pri-miR396b levels with no significant effect on the PAMP-insensitive pri-miR166a (Figure 9C). Figure 9C shows AvrPtoB suppresses PAMP-responsive pri-miRNAs accumulation. Efr1 mutant plants were Agro-infiltrated at a OD of 0.4 with 35S::GUSintron, 35S:AvrPtoB and pri-miRNA levels monitored by semi-quantitative RT-PCR analysis. Thus, AvrPtoB potentially inhibits PAMP-responsive miRNA expression at the transcriptional level.

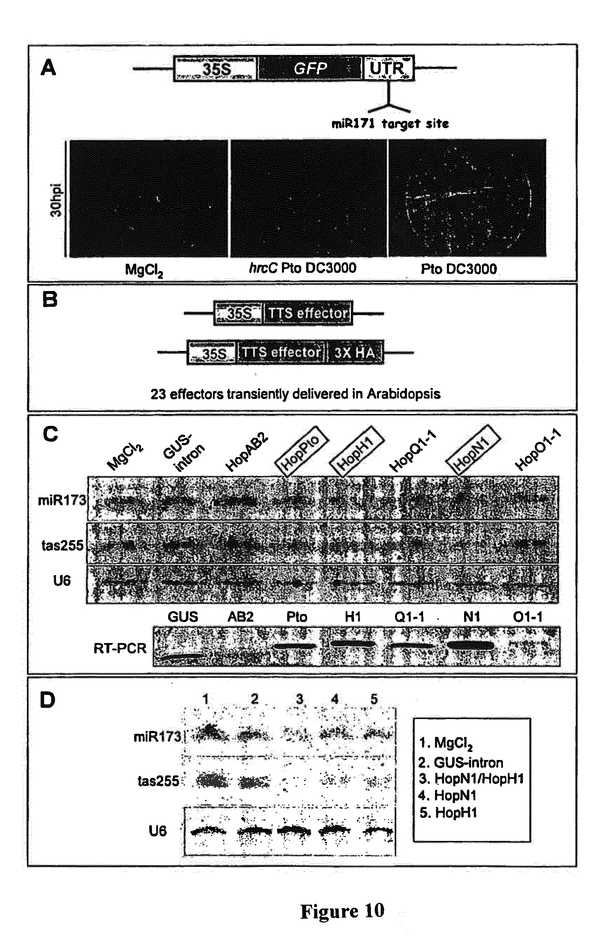

We further tested whether the overall miRNA pathway could be affected by Pst DC3000 effectors. For this purpose, we generated Col-0 transgenic plants expressing a sensor construct depicting miR171 activity. miR171, when expressed, silences a GFP reporter construct. Such transgenic plants constitutively express a *GFP* reporter gene in which a miR171 target site is added in the 3 'UTR of the *GFP* gene (Figure 10A). The miRNA target site was modified in order to avoid RDR6-triggered transgene transitivity as previously described. Because miR171 is well expressed in leaves, no GFP signal can be detected in standard growth conditions. Leaves from the miR171 sensor lines were challenged with the virulent Pst DC3000 and the non-virulent TTS-deficient *Pst* DC3000 *hrcC,* and GFP levels monitored over a timecourse experiment. We found that virulent *Pst* DC3000, but not *Pst* DC3000 *hrcC,* restored GFP gene expression at 24 hours post inoculation (Figure 10A). Figure 10A shows how Pst DC3000 restores GFP expression in the miR171 sensor lines. Schematic representation of the miR171 sensor construct (upper panel). Leaves from miR171 sensor lines were inoculated with mock (MgCl₂) (left panel), *hrcC Pst* DC3000 (middle panel) and virulent *Pst* DC3000 (right panel). Pictures were taken 30 hours post inoculation (hpi). A concentration of 10⁵ colony forming units (cfu/ml) was used in this assay. This result indicates that some TTS proteins act as suppressors of the miRNA pathway either by inhibiting the miRNA biogenesis and/or the miRNA activity.

### Example 5

### Identification of Proteins that Suppress miRNA Pathways

To identify such bacterial factors, 23 individual TTS effector genes (driven by the strong 35S promoter) were transiently delivered in *Arabidopsis efr1* mutant leaves (Figure 10B) (Figure 10B shows accumulation of both miR173 and tas255 is partially impaired in leaves expressing hopN1, H1 and Pto. The 23 constructs were transiently delivered in *Arabidopsis* leaves using *Agrobacterium* transformation and a low molecular weight Northern analysis was performed using oligo probes complementary to miR173 and tas255 small RNAs.) The levels of the endogenous miR173 and Tas255 (which relies on miR173 activity for its biogenesis) were monitored. We found that three of those proteins (the cysteine protease HopN1 HopH1, and AvrPto) caused a significant decrease in the steady-state levels of miR173 and Tas255 (Figure 10C). Figure 10C shows accumulation of miR173 and tas255 is drastically impaired in leaves co-expressing hopN1 and hopH1. A transient delivery of hopN1 and hopH1 overexpressor contructs were performed as described in Figure 10B Northern analysis was done as in Figure 10B. Moreover, these effects were additive upon co-delivery of HopN1 and HopH1 indicating that these proteins interfere, directly or indirectly, with distinct host factors involved in RNA-silencing. Both HopN1 wildtype and mutant versions, the latter being altered in the predicted cysteine catalytic triad (C172S, H283A and D299A, rescued GFP expression when delivered in transgenic *efr1* plants expressing miR171 sensor construct described above (data not shown). All the HopN1 mutant versions were stable *in planta* as assayed by Western analysis (data not shown).

Figure 10D shows HopN1-triggered suppression of RNA-silencing does not require cysteine protease activity. *Efr1* mutant plants expressing the miR171 sensor construct were agro-infiltrated (OD=0.4) with 35S::GUSintron, 35S::hopN1-HA, 35S::HopN1 C172S, 35S::HopN1 H283A and the levels of GFP analyzed visually under UV. This indicates that the cysteine protease activity is not required for suppression of RNA-silencing. All together, the identified TTS proteins suppress miRNA biogenesis. They are thus referred to here as 'Bacterial silencing suppressors' (Bss).
- The nucleotide sequences (coding sequences) from the Bss proteins that suppress miRNA biogenesis are as follows:
   >AvrPtoB coding sequence: 
ATGGCGGGTATCAATAGAGCGGACCATCGGGCGCTTATTTTGTTGGCCACACAGACCCCGAGCCAGTATC
GGGGCAAGCACACGGATCCGGCAGCGGCGCCAGCTCCTCGAACAGTCCGCAGGTTCAGCCGCGACCCTCGA
ATACTCCCCCGTCGAACGCGCCCGCACCGCCGCCAACCGGACGTGAGAGGCTTTCACGATCCACGGCGCTG
TCGCGCCAAACCAGGGAGTGGCTGGAGCAGGGTATGCCTACAGCGGAGGATGCCAGCGTGCGTCGTAGGCC
ACAGGTGACTGCCGATGCCGCAACGCCGCGTGCAGAGGCAAGACGCACGCCGGAGGCAACTGCCGATGCCA
GCGCACCGCGTAGAGGGGCGGTTGCACACGCCAACAGTATCGTTCAGCAATTGGTCAGTGAGGGCGCTGAT
ATTTCGCATACTCGTAACATGCTCCGCAATGCAATGAATGGCGACGCAGTCGCTTTTTCTCGAGTAGAACA
GAACATATTTCGCCAGCATTTCCCGAACATGCCCATGCATGGAATCAGCCGAGATTCGGAACTCGCTATCG
AGCTCCGTGGGGCGCTTCGTCGAGCGGTTCACCAACAGGCGGCGTCAGCGCCAGTGAGGTCGCCCACGCCA
ACACCGGCCAGCCCTGCGGCATCATCATCGGGCAGCAGTCAGCGTTCTTTATTTGGACGGTTTGCCCGTTT
GATGGCGCCAAACCAGGGACGGTCGTCGAACACTGCCGCCTCTCAGACGCCGGTCGACAGGAGCCCGCCAC
GCGTCAACCAAAGACCCATACGCGTCGACAGGGCTGCGATGCGTAATCGTGGCAATGACGAGGCGGACGCC
GCGCTGCGGGGGTTAGTACAACAGGGGGTCAATTTAGAGCACCTGCGCACGGCCCTTGAAAGACATGTAAT
GCAGCGCCTCCCTATCCCCCTCGATATAGGCAGCGCGTTGCAGAATGTGGGAATTAACCCAAGTATCGACT
TGGGGGAAAGCCTTGTGCAACATCCCCTGCTGAATTTGAATGTAGCGTTGAATCGCATGCTGGGGCTGCGT
CCCAGCGCTGAAAGAGCGCCTCGTCCAGCCGTCCCCGTGGCTCCCGCGACCGCCTCCAGGCGACCGGATGG
TACGCGTGCAACACGATTGCGGGTGATGCCGGAGCGGGAGGATTACGAAAATAATGTGGCTTATGGAGTGC
GCTTGCTTAACCTGAACCCGGGGGTGGGGGTAAGGCAGGCTGTTGCGGCCTTTGTAACCGACCGGGCTGAG
CGGCCAGCAGTGGTGGCTAATATCCGGGCAGCCCTGGACCCTATCGCGTCACAATTCAGTCAGCTGCGCAC
AATTTCGAAGGCCGATGCTGAATCTGAAGAGCTGGGTTTTAAGGATGCGGCAGATCATCACACGGATGACG
TGACGCACTGTCTTTTTGGCGGAGAATTGTCGCTGAGTAATCCGGATCAGCAGGTGATCGGTTTGGCGGGT
AATCCGACGGACACGTCGCAGCCTTACAGCCAAGAGGGAAATAAGGACCTGGCGTTCATGGATATGAAAAA
ACTTGCCCAATTCCTCGCAGGCAAGCCTGAGCATCCGATGACCAGAGAAACGCTTAACGCCGAAAATATCG
CCAAGTATGCTTTTAGAATAGTCCCCTGA
   >HopH1 coding sequence: 
ATGATCACTCCGTCTCGATATCCAGGCATCTATATCGCCCCCCTCAGTAACGAACCGACAGCAGCTCACAC
ATTTAAAGAACAAGCAGAGGAAGCACTTGACCATATCAGCGCCGCACCCTCTGGCGATAAGCTATTGCGAA
AAATATCCACTCTTGCCAGTCAAAAAGATAGAAAAGTCACGCTAAAAGAGATTGAAATAAATAACCAGTGT
TATACCGAAGCTGTTCTGAGCAGGAGGCAACTGGAAAAGTACGAACCAGAAAACTTTAACGAGAACCGGCA
CATTGCATCACAGCTATCACGAAAGGGGACCTTTACCAAAGGTGAAGGAAGCAACGCGATTATTGGCTGGT
CACCAGACAAAGCAAGCATACGCTTAAATCAGAATGGCTCACCGTTACACCTTGGAATGGATAACGACGAC
AAAATCACGACCCTAGCTCATGAGCTCGTTCATGCTCGACATGTGTTAGGTGGCAGCTCCTTAGCGGATGG
CGGAGATCGCTATAATCCACGTACGGGATCTGGCAAAGAGGAACTTAGGGCCGTTGGATTAGATAAGTACC
GCTATTCACTTACAAAAAAACCGTCAGAGAACTCCATCCGAGCTGAACACGGCCTGCCTCTGCGCATGAAG
TACAGGGCACATCAATAG
   >HopN1 coding sequence: 
ATGTATATCCAGCAATCTGGCGCCCAATCAGGGGTTGCCGCTAAGACGCAACACGATAAGCCCTCGTCATT
GTCCGGACTCGCCCCCGGTTCGTCGGATGCGTTCGCCCGTTTTCATCCCGAAAAGGCGGCGCCTTTGTCC
CATTGGAGGGGCATGAAGaGGTCTTTTTCGATGCGCGCTCTTCCTTTTCGTCGGTCGATGCCGCTGATCTT
CCCAGTCCCGAGCAGGTACAACCCCAGCTTCATTCGTTGCGTACCCTGCTACCGGATCTGATGGTCTCTAT
CGCCTCATTACGTGACGGCGCCACGCAATACATCAAGACCAGAATCAAGGCTATGGCGGACAACAGCATAG
GCGCGACTGCGAACATCGAAGCCAAAAGAAAGATTGCCCAAGAGCACGGCTGTCAGCTTGTCCACCCGTTT
CACCAGAGCAAATTTCTATTTGAAAAAACTATCGATGATAGAGCGTTTGCTGCTGATTATGGCCGCGCGGG
TGGCGACGGGCACGCTTGTCTGGGGCTATCAGTAAATTGGTGTCAGAGCCGTGCAAAAGGGCAGTCGGATG
AGGCCTTCTTTCACAAACTGGAGGACTATCAGGGCGATGCATTGCTACCCAGGGTAATGGGCTTCCAGCAT
ATCGAGCAGCAGGCCTATTCAAACAAGTTGCAGAACGCAGCACCTATGCTTCTGGACACACTTCCCAAGTT
GGGCATGACACTTGGAAAAGGGCTGGGCAGAGCACAGCACGCGCACTATGCGGTTGCTCTGGAAAACCTTG
ATCGCGATCTCAAAGCAGTGTTGCAGCCCGGTAAAGACCAGATGCTTCTGTTTTTGAGTGATAGCCATGCG
ATGGCTCTGCATCAGGACAGTCAGGGATGTCTGCATTTTTTTGATCCTCTTTTTGGCGTGGTTCAGGCAGA
CAGCTTCAGCAACATGAGCCATTTTCTTGCTGATGTGTTCAAGCGCGACGTAGGTACGCACTGGCGTGGCA
CGGAGCAACGTCTGCAACTGAGCGAAATGGTGCCCAGAGCAGACTTTCACTTGCGATAA
   >AvrPto coding sequence: 
ATGGAAATATATGTGTCGGCGGATCCAGGATGGCCCATCAGGTGAACTCCCCAGACCGAGTTAGTAACAA
CTCGGGTGACGAAGATAACGTAACGTCCAGTCAACTGCTGAGCGTCAGACATCAACTTGCGGAGTCTGCTG
GTGTACCAAGAGATCAGCATGAATTTGTTAGTAACCAAGCACCTCAAAGCCTGAGAAATCGCTACAACAAT
CTTTACTCACATACGCAAAGAACACTGGATATGGCGGACATGCAGCATAGGTACATGACGGGAGCGTCAGG
AATCAATCCGGGAATGCTGCCACATGAGAATGTGGACGATATGCGTAGCGCTATAACTGATTGGAGTGACA
TGCGCGAAGCTCTGCAGTACGCAATGGGTATCCATGCCGACATCCCACCGTCTCCAGAGCGATTTGTTGCG
ACTATGAACCCGAACGGATCAATTCGAATGTCAACACTTTCTCCTAGCCCGTACCGTAACTGGCAATGA
- The amino acid sequences from the Bss proteins that suppress miRNA biogenesis are as follows:
   >HopPtoB amino acid sequence: 
MAGINRAGPSGAYFVGHTDPEPVSGQAHGSGSGASSSNSPQVQPRPSNTPPSNAPAPPPTGRERLSRSTAL
SRQTREWLEQGMPTAEDASVRRRPQVTADAATPRAEARRTPEATADASAPRRGAVAHANSIVQQLVSEGAD
ISHTRNMLRNAMNGDAVAFSRVEQNIFRQHFPNMPMHGISRDSELAIELRGALRRAVHQQAASAPVRSPTP
TPASPAASSSGSSQRSLFGRFARLMAPNQGRSSNTAASQTPVDRSPPRVNQRPIRVDRAAMRNRGNDEADA
ALRGLVQQGVNLEHLRTALERHVMQRLPIPLDIGSALQNVGINPSIDLGESLVQHPLLNLNVALNRMLGLR
PSAERAPRPAVPVAPATASRRPDGTRATRLRVMPEREDYENNVAYGVRLLNLNPGVGVRQAVAAFVTDRAE
RPAVVANIRAALDPIASQFSQLRTISKADAESEELGFKDAADHHTDDVTHCLFGGELSLSNPDQQVIGLAG
NPTDTSQPYSQEGNKDLAFMDMKKLAQFLAGKPEHPMTRETLNAENIAKYAFRIVP
   >HopH1 amino acid sequence: 
MITPSRYPGIYIAPLSNEPTAAHTFKEQAEEALDHISAAPSGDKLLRKISTLASQKDRKVTLKEIEINNQC
YTEAVLSRRQLEKYEPENFNENRHIASQLSRKGTFTKGEGSNAIIGWSPDKASIRLNQNGSPLHLGMDNDD
KITTLAHELVHARHVLGGSSLADGGDRYNPRTGSGKEELRAVGLDKYRYSLTKKPSENSIRAEHGLPLRMK
YRAHQ
   >HopN1 amino acid sequence: 
MYIQQSGAQSGVAAKTQHDKPSSLSGLAPGSSDAFARFHPEKAGAFVPLEGHEEVFFDARSSFSSVDAADL
PSPEQVQPQLHSLRTLLPDLMVSIASLRDGATQYIKTRIKAMADNSIGATANIEAKRKIAQEHGCQLVHPF
HQSKFLFEKTIDDRAFAADYGRAGGDGHACLGLSVNWCQSRAKGQSDEAFFHKLEDYQGDALLPRVMGFQH
IEQQAYSNKLQNAAPMLLDTLPKLGMTLGKGLGRAQHAHYAVALENLDRDLKAVLQPGKDQMLLFLSDSHA
MALHQDSQGCLHFFDPLFGVVQADSFSNMSHFLADVFKRDVGTHWRGTEQRLQLSEMVPRADFHLR
   >AvrPto amino acid sequence:

MGNICVGGSRMAHQVNSPDRVSNNSGDEDNVTSSQLLSVRHQLAESAGVPRDQHEFVSNQAPQSLRNRYNN
LYSHTQRTLDMADMQHRYMTGASGINPGMLPHENVDDMRSAITDWSDMREALQYAMGIHADIPPSPERFVA
TMNPNGSIRMSTLSPSPYRNWQ

### Example 6

### Identification of Proteins that Suppress AGO1-Containing RISC

We then investigated whether any Pst DC3000 effectors could also suppress the AGO1-containing RISC function as observed with some viral-derived suppressors of RNA-silencing. The same set of 23 Pto DC3000 effectors was further tested for possible interference with miRNA activities. Transient expression of one or more HopT1-1 did not affect miR834 accumulation but dramatically increased the levels of its cognate target, the COP-interacting protein 4 (CIP4) (Figure 11A). By contrast, neither miR834 nor CIP4 levels were altered upon transient delivery of the unrelated Pto DC3000 effectors HopC1, -X1 (Figure 11A).

Figure 11A shows overexpression of hopT1-1 promotes the accumulation of miR834 target and has no significant effect on miR834 steady state levels. *Efr1* mutant leaves were agro-infiltrated with 35S::hopT1-1 construct (OD=0.4) and miR834 as well as miR834 target (COP-interacting protein 4, CIP4) levels were monitored 3 days post infiltration by Northern analysis (Upper panel) and Western analysis (Bottom panel), respectively.

These results suggested that HopT1-1 acts downstream of miRNA biogenesis, potentially by inhibiting the AGO1-directed RISC, which is recruited by most plant miRNAs.

To test this hypothesis further, we used the SUC-*SUL* (SS) reporter line 120 in which phloem-specific expression of an inverted-repeat transgene triggers non-cell-autonomous RNAi of the endogenous SULPHUR (SUL) transcript, resulting in a chlorotic phenotype that expands beyond the vasculature (Figure 11B). Figure 11B shows a weak allele of *ago1* suppresses artificial silencing with no significant effect on the steady state levels of 21-24 nt siRNAs. *Ago1-12* mutation suppresses artificial silencing triggered by an artificial hairpin, targeting the endogene sulphure (SUL), and driven by the phloem specific promoter (SUC2). *Ago1-12* suppresses the vein chlorotic phenotype triggered by SUC-SUL transgene (Upper panel). *Ago1-12* mutation does not significantly interfere with the accumulation of 21-24 nt siRNA as assayed by Northern analysis (bottom panel). Of the 21nt (DCL4-dependent) and 24nt (DCL3-dependent) *SUL* siRNA species, only the former is required for RNAi in a strict AGO1-dependent manner.

The 35S::HopT1-1 construct was transformed into SUC-*SUL*, and two independent T2 lines were selected, showing moderate (#4) or strong (#22) HopT1-1 mRNA accumulation (Figure 11C). Figure 11C shows overexpression of HopT1-1 suppresses the SUC-SUL phenotype with no significant effect on neither 21-24 nt siRNA nor endogenous miRNA accumulation. Suppression of the vein chlorotic phenotype trigger by the SUC-SUL transgene (Upper panel). Low molecular weight Northern on the 21-24 SUL siRNA (middle and right panel), RT-PCR on HopT1 mRNA (middle and left panel), LMW Northern on a subset of endogenous miRNAs (Bottom panel). Both lines exhibited little or no cholorosis and accumulated higher *SUL* mRNA levels. However, accumulation of *SUL* siRNAs remained unaltered mimicking the effects of the *ago1-12* mutation in SUC-*SUL* plants. Also as in *ago1-12* mutants, the levels of several canonical miRNAs were normal in HopT1-1-expressing lines, despite higher accumulation of their target transcripts.

Figure 11D shows schematic representation depicting the expected effect of a perturbation of the RISC^{miRNA} function. Figure 11E shows overexpression of hopT1-1 promotes the accumulation of miR834 target (CIP4) with no effect on miR834 accumulation. Western analysis results using an anti-CIP4 antibody. Figure 11F shows overexpression of hopT1-1 promotes the accumulation of miRNA targets. A subset of miRNA targets are more elevated in two independent transgenic lines overexpressing hopT1-1 (Left panel). The same subset of miRNA targets are more elevated in *ago1-11* and *ago1-12* mutants (Right panel). Collectively these results indicate that HopT1-1 likely interferes with the function of the AGO1-RISC, resulting in suppression of miRNA, as well siRNA activities.

We obtained similar results when we overexpressed hopY1 effector in the silencing reporter line SUC-SUL (Figure 12), except that a slight increase in the levels of 21-24 nt SUL siRNA as well as endogenous miRNAs was observed in this particular case. HopY1 overexpressor lines displayed a significant increase in the accumulation of several miRNA targets (Figure 12C). Figure 12C shows overexpression of hopY1 slightly increases the accumulation of some endogenous miRNAs. However, despite its effect on miRNA activity, HopY1 did not induce drastic developmental alteration (Figure 12A). Figure 12A shows overexpression of hopY1 reduces the vein chlorotic phenotype triggered by the SUC-SUL transgene. Figure 12B shows overexpression of hopY1 slightly increases the accumulation of 21-24 nt SUL siRNAs. Figure 12D shows overexpression of hopY1 promotes the accumulation of a subset of miRNA targets as well as SUL mRNA.

The nucleotide sequences encoding the Bss proteins that interfere with the RISC^{miRNA} function are as follows:
>HopT1-1 coding sequence: 
ATGATGAAAACAGTCAGCAATCACTCGATACCCAGTACAAATCTCGTCGTGGATGCGGGAACGGAAACTTC
GGCGCAGAAATCCCAGCCGGTTTGCAGCGAAATCCAGCGTAACAGCAAGATCGAAAAAGCAGTCATCGAAC
ACATTGCCGACCACCCGGCAGCGAAAATGACAATAAGCGCGCTGGTTGACACGTTGACAGACGTTTTTGTC
AGGGCTCATGGGGAGGTTAAGGGGTGGCCGAAATCGTCCAGGCAGTCTCTCGCCCTCATGACAGTAATCG
ACACGGCAGTGGAGTGCTCAGCCCGCGCTTTGATGTAATGGGGAGTGTTGGTTGGAATGCGGCAGCTATCC
GGGCCACCAGTCGCGTCGGGACGCTTCGAGAGAAAGGTACACTGTTCACTAACCTTATGCTCAGTAACAAC
TTTAAACATTTGCTTAAACGAGTGGTTAACGATCCAGCCTTGCAGCAAAAGCTCGACGGTGGGTTAGACCT
CAACTATCTGAAGGCTTGTGAAGGCGATCTTTATGTCATGTCAGGGTGGGCTGCACGGGCTAGCGAAAGTC
GTGAACAAATTGGCAAAGCCCGGTATGAAACGGCATCAAATCTTAGCCAGACGCTGATCAGTGCACGTGAG
TTGGCTTTTCATCGTCACAATCCGGTTAATCATCCGTCTGCCCAAACGAAAGTGGGCTTCGATAAGGGTTT
GCCTGAGGAATCTGATCTGCAGGTTCTGAGAGGCCATGGCAGCAGTGTATGGAGTGTAAAACCGGGCAGCG
ATTTCGCAAAGCGTGCTGAAGTTTCTGGAAAGCCTATTATCGCCGGCCCGTCCGGTACCGCTTCGCGCATG
GTCGCTGTTGCGCGTTTTCTGGCACCGGCTTGTTTGAAAAGCCTGGGTATTGAGAGTGAGCAGAACCTGAA
AGAGCTTGTGCGGTATGCCTGCTATGCCTATTTCGGTCAGGACAGCCACCATTCGATGCTTGAAGTGAATC
TTGGTGTCGCTTCCCATGGAATGCCGGAACAATGGACGACACGCTTTATAACGAGCCTTTCAGTAATTCA
ATTAAAGGTCGCGGGTTTGGTATAGACAATCTCGCGCATAGGCAAGTCGTCAGGCAGGCGGCTCAAAAGTC
ATGA
>HopY1 coding sequence :

ATGAACATTACGCCGCTCACGTCAGCCGCGGGCAAGGGCTCGTCCGCACAAGGCACAGACAAAATTTCCAT
TCCCAACTCCACGCGCATGATCAATGCCGCTTCAATCAAGTGGTTGAATAAGGTGCGTAGCGCCATCAGTG
ACCACATCCGCACCAGCATCGAGAAAGGGAAACTGTTCGAGCTCGCCTCCTTGGGCAGCAACATGTTCGGT
GTCCCGGCTCTTTCAGCGCGCCCCTCGACGCTCCAACCTGTGTTGGCGTTTGAGGCTGACCCCAATCACGA
CCTGAACCTTGTCAGGGTCTATATGCAGGACAGCGCCGGCAAGCTCACTCCCTGGGACCCGACGCCCAACG
CGGTCACGACGACGTCGAATCCATCAGAGCCTGATGCGCAGAGCGATACGGCTTCGTCATCATTACCTCGG
CGGCCTCCCGCAGGCTCGGTGCTGAGTTTGCTGGGCATTGCGCTGGATCACGCGCAACGCCACAGTCCTCG
CGCGGACAGGTCTGCCAAGGGACGACCTGGCCGAGAGGAGAGGAACGGGGCAAGGTTCAATGCCAAGCAAA
CAAAGCCGACAGAGGCTGAAGCCTACGGTGATCATCAGACACCCAATCCTGATTTGCACAGGCAAAAAGAG
ACAGCTCAACGCGTTGCTGAAAGCATCAACAGCATGCGAGAGCAGCAAAATGGAATGCAACGCGCCGAAGG
GCTTCTCAGAGCCAAAGAAGCGTTGCAAGCTCGGAAGCCGCGCGCAAGCAGCTTCTGGACGTGCTCGAGG
CCATCCAGGCTGGCCGTGAAGACTCCACCGACAAGAAGATCAGCGCCACTGAAAAGAACGCCACGGGCATC
AACTACCAGTGA

The amino acid sequences from the Bss proteins that interfere with the RISC^{miRNA} function are as follows:
>HopT1-1 amino acid sequence : 
MMKTVSNHSIPSTNLVVDAGTETSAQKSQPVCSEIQRNSKIEKAVIEHIADHPAAKMTISALVDTLTDVFV
RAHGEVKGWAEIVQAVSRPHDSNRHGSGVLSPRFDVMGSVGWNAAAIRATSRVGTLREKGTLFTNLMLSNN
FKHLLKRVVNDPALQQKLDGGLDLNYLKACEGDLYVMSGWAARASESREQIGKARYETASNLSQTLISARE
LAFHRHNPVNHPSAQTKVGFDKGLPEESDLQVLRGHGSSVWSVKPGSDFAKRAEVSGKPIIAGPSGTASRM
VAVARFLAPACLKSLGIESEQNLKELVRYACYAYFGQDSHHSMLEVNLGVASHGMPEQWDDTLYNEPFSNS
IKGRGFGIDNLAHRQVVRQAAQKS
>HopY1 amino acid sequence:

MNITPLTSAAGKGSSAQGTDKISIPNSTRMINAASIKWLNKVRSAISDHIRTSIEKGKLFELASLGSNMFG
VPALSARPSTLQPVLAFEADPNHDLNLVRVYMQDSAGKLTPWDPTPNAVTTTSNPSEPDAQSDTASSSLPR
RPPAGSVLSLLGIALDHAQRHSPRADRSAKGRPGREERNGARFNAKQTKPTEAEAYGDHQTPNPDLHRQKE
TAQRVAESINSMREQQNGMQRAEGLLRAKEALQAREAARKQLLDVLEAIQAGREDSTDKKISATEKNATGI
NYQ

### Example 7

### Identification of Proteins that Suppress miRNA Translational Inhibition

We finally investigated whether bacterial effector proteins could suppress miRNA-directed translational inhibition, a phenomenon well characterized in animals but also effective in plants. For this purpose, we transiently delivered the same set of Pst DC3000 effectors in the *efr1* mutant leaves and screened for effectors that would enhance protein accumulation of miRNA targets with no major impact on either miRNA target mRNA levels nor mature miRNA levels. Among the subset of effectors tested, the mono-ADP ribosyltransferase HopU1 fulfilled these criteria as it induces higher miR398 target protein levels: the Superoxide dismutase 1 (CSD1) and 2 (CSD2), with no significant effect on the accumulation of CSD1/2 mRNA levels or miR398 levels (data not shown). Moreover, delivery of HopU1 in the *efr1* mutant expressing the miR171 sensor construct restored a high GFP protein accumulation but did not alter GFP mRNA levels indicating that HopU1 indeed interferes with miRNA-directed translational inhibition (Figure 13A/B and data not shown). Figure 13A shows overexpression of HopU1 but not HopUIDD mutant version restores GFP expression in efr1 plants expressing miR171 sensor constructs. miR171 Sensor (efr1) plants were Agro-infiltrated (OD=0.4) with the 35S::GUSintron, 35S::HopU1 and 35S::HopU1DD constructs and GFP levels analyzed visually under UV. Figure 13B shows Western analysis using an anti-GFP antibody. Importantly, no rescue of GFP expression nor of the endogenous miR398 target CSD2 was observed when we transiently delivered a HopU1 mutant version that abolishes its ADP-ribosyltransferase activity. This indicates that ADP-ribosyltransferase activity is required for HopU1-triggered suppression of RNA-silencing.

To further investigate whether HopU1 interferes with a putative siRNA-directed translational inhibition, we transformed 35S::HopU1 construct in the SUC-*SUL* reference line and selected T2 transgenic lines expressing high levels of hopU1 proteins. Expression of HopU1 in these stable transgenic lines diminishes slightly the 21-24 SUL siRNA levels, but did not affect SUL mRNA levels. Figure 13C shows SUC-SUL plants expressing HopU1.

Figure 13D shows LMW Northern analysis. However, a significant reduction in the vein chlorotic phenotype was observed, which is diagnostic of higher SUL protein levels. Western analysis using an anti CSD2 antibody suggests that HopU1 additionally interferes with siRNA-directed translational inhibition. Figure 13E shows qRT-PCR on SUL mRNA.

The nucleotide sequence encoding the Bss protein interferes with miRNA-mediated translational inhibition is as follows:
>HopU1 coding sequence:

ATGAATATAAATCGACAACTGCCTGTATCAGGCTCGGAGCGATTGTTGACTCCCGACGTGGCGTATCTCG
CCAGGCTTGTTCCGAAAGGCATTATTCTACTGGACAGGATCGGCATGATTTTTACCGTTTTGCTGCCAGGC
TACATGTGGATGCGCAGTGTTTTGGTCTGTCAATAGACGATTTGATGGATAAGTTTTCTGACAAGCACTTC
AGGGCTGAGCATCCTGAATACAGGGATGTCTATCCGGAGGAATGTTCTGCCATTTATATGCATACCGCTCA
AGACTATTCTAGTCACCTCGTAAGGGGGGAAATAGGAACGCCGCTGTACCGAGAGGTCAATAATTATCTTC
GACTTCAACATGAGAATTCTGGCGAGAAGCTGAAATTGATAATCACGACGAAAAGCTATCGCCTCACATA
AAAATGCTTTCATCTGCGCTTAATCGTTTAATGGATGTCGCCGCTTTTAGAGGAACGGTTTATAGAGGCAT
TCGCGGTGATTTAGATACCATTGCTCGGCTCTACCATCTATTCGATACGGGCGGCCGGTACGTAGAGCCCG
CTTTCATGAGTACAACTCGAATAAAGGACAGTGCCCAGGTGTTTGAGCCAGGCACGCCAAACAACATAGCT
TTCCAGATAAGCCTAAAAAGAGGCGCCGACATTTCGGGATCTTCCCAAGCGCCCTCAGAGGAAGAAATCAT
GCTACCCATGATGAGTGAGTTCGTCATTGAACATGCATCCGCTCTTTCCGAAGGAAAGCATTTATTTGTAT
TAAGTCAGATTTGA

The amino acid sequence of the Bss protein that interferes with miRNA-mediated translational inhibition is as follows:
>HopU1 amino acid sequence:

MNINRQLPVSGSERLLTPDVGVSRQACSERHYSTGQDRHDFYRFAARLHVDAQCFGLSIDDLMDKFSDKHF
RAEHPEYRDVYPEECSAIYMHTAQDYSSHLVRGEIGTPLYREVNNYLRLQHENSGREAEIDNHDEKLSPHI
KMLSSALNRLMDVAAFRGTVYRGIRGDLDTIARLYHLFDTGGRYVEPAFMSTTRIKDSAQVFEPGTPNNIA
FQISLKRGADISGSSQAPSEEEIMLPMMSEFVIEHASALSEGKHLFVLSQI

Bss proteins mutated in key residues that do not perturb suppression of RNA-silencing will be particularly useful as they might not be recognized by plant resistance (R) proteins and therefore would not induce the classical R-mediated programmed cell death (which is often detrimental for the plants). Examples include versions of HopN1 that are mutated in the predicted cysteine protease catalytic triads and still retained their ability to suppress RNA-silencing (Figure 10C). These mutant versions might be compromised in R gene recognition and yet still suppress RNA-silencing in different plant species. The method hereby disclosed thus allows the generation of mutated versions of Bss proteins in order to uncouple the suppression of RNA-silencing from the R-gene recognition.

Bss identified according to this invention are also useful proteins to identify plant and animal components involved in miRNA biogenesis and/or activity. As an example, the mono-ADP-ribosyltransferase HopU1 discussed above was recently shown to directly interact with the glycine-rich RNA-binding proteins AtGRP7 and AtGRP8. ADP-ribosylation of GRP7 by HopU1 occurs on two conserved arginine residues located in the RNA-recognition domain of GRP7 and likely perturbs its ability to bind RNA. Because HopU1 ADP-ribosyltransferase activity is required for its RNA-silencing suppression activity, we anticipate that AtGRP7 and AtGRP8 are novel silencing factors involved in miRNA- and siRNA-directed translational inhibition. Figure 13 shows overexpression of HopU1 but not HopU1DD mutant version restore GFP expression in efr1 plants expressing miR171 sensor constructs. Other Bss proteins will also directly interact with and perturb host components involved in RNA-silencing and can therefore be used as molecular probes to identify RNA-silencing factors or regulators thereof, in both plant and animal systems.

The coding sequences from the putative novel RNA-silencing components from *Arabidopsis thaliana* are as follows:
>AtGRP7 (At2g21660) coding sequence: 
ATGGCGTCCGGTGATGTTGAGTATCGGTGCTTCGTTGGAGGTCTAGCATGGGCCACTGATGACAGAGCTCT
TGAGACTGCCTTCGCTCAATACGGCGACGTTATTGATTCCAAGATCATTAACGATCGTGAGACTGGAAGAT
CAAGGGGATTCGGATTCGTCACCTTCAAGGATGAGAAAGCCATGAAGGATGCGATTGAGGGAATGAACGGA
CAAGATCTCGATGGCCGTAGCATCACTGTTAACGAGGCTCAGTCACGAGGAAGCGGTGGCGGCGGAGGCCA
CCGTGGAGGTGGTGGCGGTGGATACCGCAGCGGCGGTGGTGGAGGTTACTCCGGTGGAGGTGGTAGCTACG
GAGGTGGCGGCGGTAGACGCGAGGGTGGAGGAGGATACAGCGGCGGCGGCGGCGGTTACTCCTCAAGAGGT
GGTGGTGGCGGAAGCTACGGTGGTGGAAGACGTGAGGGAGGAGGAGGATACGGTGGTGGTGAAGGAGGAGG
TTACGGAGGAAGCGGTGGTGGTGGAGGATGGTAA
>AtGRP8 (At4g39260) coding sequence:

ATGTCTGAAGTTGAGTACCGGTGCTTTGTCGGCGGCCTTGCCTGGGCCACCAATGATGAAGATCTTCAAAG
GACGTTCTCACAGTTCGGCGACGTTATCGATTCTAAGATCATTAACGACCGCGAGAGTGGAAGATCAAGGG
GATTCGGATTCGTCACCTTCAAGGACGAGAAAGCCATGAGGGATGCGATTGAAGAGATGAACGGTAAAGAG
CTCGATGGACGTGTCATCACCGTGAACGAGGCTCAGTCGAGAGGTAGCGGCGGTGGCGGAGGAGGCCGTGG
TGGAAGCGGTGGTGGTTACCGCAGCGGAGGCGGTGGTGGATACTCAGGAGGCGGTGGCGGCGGATACTCAG
GAGGAGGCGGTGGTGGTTACGAGAGACGTAGCGGAGGTTACGGATCTGGTGGAGGCGGTGGTGGCCGAGGA
TACGGTGGTGGTGGACGCCGTGAGGGAGGTGGCTACGGAGGCGGTGATGGTGGAAGTTACGGAGGCGGTGG
TGGCGGCTGGTAA

The amino acid sequences from the putative novel RNA-silencing components from *Arabidopsis* are as follows:
>AtGRP7 amino acid sequence: 
MASGDVEYRCFVGGLAWATDDRALETAFAQYGDVIDSKIINDRETGRSRGFGFVTFKDEKAMKDAIEGMNG
QDLDGRSITVNEAQSRGSGGGGGHRGGGGGGYRSGGGGGYSGGGGSYGGGGGRREGGGGYSGGGGGYSSRG
GGGGSYGGGRREGGGGYGGGEGGGYGGSGGGGGW
>AtGRP8 amino acid sequence:

MSEVEYRCFVGGLAWATNDEDLQRTFSQFGDVIDSKIINDRESGRSRGFGFVTFKDEKAMRDAIEEMNGKE
LDGRVITVNEAQSRGSGGGGGGRGGSGGGYRSGGGGGYSGGGGGGYSGGGGGGYERRSGGYGSGGGGGGRG
YGGGGRREGGGYGGGDGGSYGGGGGGW

Homology search in animals revealed several possible orthologs of AtGRP8 in humans, suggesting that one or several of these orthologs might be involved in executing or regulating miRNA-directed functions in animals. Therefore, plant and animal Bss are also valuable tools to uncover novel RNA silencing components in a broad range of organisms. The various human proteins with homology to AtGR8 are listed below:
>HsCIRBP gi|4502847|ref|NP_001271.1| cold inducible RNA binding protein [Homo sapiens] 
MASDEGKLFVGGLSFDTNEQSLEQVFSKYGQISEVVVVKDRETQRSRGFGFVTFENIDDAKDAMMAMNGKS
VDGRQIRVDQAGKSSDNRSRGYRGGSAGGRGFFRGGRGRGRGFSRGGGDRGYGGNRFESRSGGYGGSRDYY
SSRSQSGGYSDRSSGGSYRDSYDSYATHNE
>HsCIRP CDS: 
ATGGCATCAGATGAAGGCAAACTTTTTGTTGGAGGGCTGAGTTTTGACACCAATGAGCAGTCGCTGGAGCA
GGTCTTCTCAAAGTACGGACAGATCTCTGAAGTGGTGGTTGTGAAAGACAGGGAGACCCAGAGATCTCGGG
GATTTGGGTTTGTCACCTTTGAGAACATTGACGACGCTAAGGATGCCATGATGGCCATGAATGGGAAGTCT
GTAGATGGACGGCAGATCCGAGTAGACCAGGCAGGCAAGTCGTCAGACAACCGATCCCGTGGGTACCGTGG
TGGCTCTGCCGGGGGCCGGGGCTTCTTCCGTGGGGGCCGAGGACGGGGCCGTGGGTTCTCTAGAGGAGGAG
GGGACCGAGGCTATGGGGGGAACCGGTTCGAGTCCAGGAGTGGGGGCTACGGAGGCTCCAGAGACTACTAT
AGCAGCCGGAGTCAGAGTGGTGGCTACAGTGACCGGAGCTCGGGCGGGTCCTACAGAGACAGTTACGACAG
TTACGCTACACACAACGAGTAA
>HsRBP3 gi|5803137|ref|NP_006734.1| RNA binding motif protein 3 [Homo sapiens] 
MSSEEGKLFVGGLNFNTDEQALEDHFSSFGPISEVVVVKDRETQRSRGFGFITFTNPEHASVAMRAMNGES
LDGRQIRVDHAGKSARGTRGGGFGAHGRGRSYSRGGGDQGYGSGRYYDSRPGGYGYGYGRSRDYNGRNQGG
YDRYSGGNYRDNYDN
>HsRBP3 CDS: 
ATGTCCTCTGAAGAAGGAAAGCTCTTCGTGGGAGGGCTCAACTTTAACACCGACGAGCAGGCACTGGAAGA
CCACTTCAGCAGTTTCGGACCTATCTCTGAGGTGGTCGTTGTCAAGGACCGGGAGACTCAGCGGTCCAGGG
GTTTTGGTTTCATCACCTTCACCAACCCAGAGCATGCTTCAGTTGCCATGAGAGCCATGAACGGAGAGTCT
CTGGATGGTCGTCAGATCCGTGTGGATCATGCAGGCAAGTCTGCTCGGGGAACCAGAGGAGGTGGCTTTGG
GGCCCATGGGCGTGGTCGCAGCTACTCTAGAGGTGGTGGGGACCAGGGCTATGGGAGTGGCAGGTATTATG
ACAGTCGACCTGGAGGGTATGGATATGGATATGGACGTTCCAGAGACTATAATGGCAGAAACCAGGGTGGT
TATGACCGCTACTCAGGAGGAAATTACAGAGACAATTATGACAACTGA
>HsRBPX gi|89059830|ref|XP_933552.1| PREDICTED: similar to RNA binding motif protein, X-linked [Homo sapiens]
>HsRBPX CDS:

ATGGGTGAAGCAGATCGCCTAGGAAAGTTTTTCATTGGTGGCTTAATACGGAAACAAATAAGAAAGCTCT
TGAAGCAGTATTTGGCAAATATGGACAAATAGTGGAAGTACACTTGATGAAAGACTGTGAAACCAACAAAT
CAAGAGGATTTGCTTTTATCACCTTTGAAAGACCAGCAGACGCTAAGGATGCAGCCAGAGACATGAATGGA
AAGTCATTAGATGGAAAAGCCATCAAGGTGGAACAAGCCACCAAACCGTCATTTGAAAGTGGTAGACGTGG
ACCGCCTCCACCTCCAAGAAGTAGAGGCCCTCCAAGAGTTCTTAGAGGTGGAAGAGGAGGAAGTGGAGGAA
CCAGGGAACCTCCCTCACGGGGAGGACACATGGATGACTGGTGGATATTCCATGAATTTTAA

### REFERENCES

1. Bernstein, E., et al. (2001) Role for a bidentate ribonuclease in the initiation step of RNA interference. Nature 409, 363-366
2. Song, J.J., et al. (2004) Crystal structure of Argonaute and its implications for RISC slicer activity. Science 305, 1434-1437
3. Liu, J., et al. (2004) Argonaute2 is the catalytic engine of mammalian RNAi. Science 305, 1437-1441
4. Rivas, F.V., et al. (2005) Purified Argonaute2 and an siRNA form recombinant human RISC. Nat Struct Mol Biol 12, 340-349
5. Napoli, C., et al. (1990) Introduction of a chimeric chalcone synthase gene into Petunia results in reversible co-suppression of homologous genes in trans. Plant Cell 2, 279-289
6. Stam, M., et al. (1997) Post-transcriptional silencing of chalcone synthase in Petunia by inverted transgene repeats. Plant Journal 12, 63-82
7. Metzlaff, M., et al. (1997) RNA-mediated RNA degradation and chalcone synthase A silencing in petunia. Cell 88, 845-854
8. Waterhouse, P.M., et al. (1998) Virus resistance and gene silencing in plants can be induced by simultaneous expression of sense and antisense RNA. Proceedings Of The National Academy Of Sciences Of The United States Of America 95, 13959-13964
9. Chuang, C.-H., and Meyerowitz, E.M. (2000) Specific and heritable genetic interference by double-stranded RNA in Arabidopsis thaliana. Proc. Natl. Acad. Sci. USA 97, 4985-4990
10. Hamilton, A.J., et al. (2002) Two classes of short interfering RNA in RNA silencing. EMBO Journal 21, 4671-4679
11. Zilberman, D., et al. (2003) ARGONAUTE4 control of locus-specific siRNA accumulation and DNA and histone methylation. Science 299, 716-719.
12. Gasciolli, V., et al. (2005) Partially Redundant Functions of Arabidopsis DICER-like Enzymes and a Role for DCL4 in Producing trans-Acting siRNAs. Curr Biol
13. Xie, Z., et al. (2005) DICER-LIKE 4 functions in trans-acting small interfering RNA biogenesis and vegetative phase change in Arabidopsis thaliana. Proc Natl Acad Sci U S A 102, 12984-12989
14. Dunoyer, P., et al. (2005) DICER-LIKE 4 is required for RNA interference and produces the 21-nucleotide small interfering RNA component of the plant cell-to-cell silencing signal. Nat Genet 37, 1356-1360
15. Dalmay, T., et al. (2000) An RNA-dependent RNA polymerase gene in Arabidopsis is required for posttranscriptional gene silencing mediated by a transgene but not by a virus. Cell 101, 543-553
16. Mourrain, P., et al. (2000) Arabidopsis SGS2 and SGS3 genes are required for posttranscriptional gene silencing and natural virus resistance. Cell 101, 533-542
17. Gazzani, S., et al. (2004) A link between mRNA turnover and RNA interference in Arabidopsis. Science
18. Glazov, E., et al. (2003) A gene encoding an RNase D exonuclease-like protein is required for post-transcriptional silencing in Arabidopsis. Plant J 35, 342-349
19. Boutet, S., et al. (2003) Arabidopsis HEN1: a genetic link between endogenous miRNA controlling development and siRNA controlling transgene silencing and virus resistance. Curr Biol 13, 843-848
20. Dalmay, T.D., et al. (2001) SDE3 encodes an RNA helicase required for post-transcriptional gene silencing in Arabidopsis. EMBO Journal 20, 2069-2078
21. Motamedi, M.R., et al. (2004) Two RNAi complexes, RITS and RDRC, physically interact and localize to noncoding centromeric RNAs. Cell 119, 789-802
22. Tomari, Y., et al. (2004) RISC assembly defects in the Drosophila RNAi mutant armitage. Cell 116, 831-841
23. Ketting, R., et al. (1999) mut-7 of C. elegans, required for transposon silencing and RNA interference, is a homolog of Werner syndrome helicase and RNaseD. Cell 99, 133-141
24. Li, J., et al. (2005) Methylation protects miRNAs and siRNAs from a 3'-end uridylation activity in Arabidopsis. Curr Biol 15, 1501-1507
25. Fagard, M., et al. (2000) AGO1, QDE-2, and RDE-1 are related proteins required for post-transcriptional gene silencing in plants, quelling in fungi, and RNA interference in animals. Proc. Natl. Acad. Sci. USA 97, 11650-11654
26. Morel, J.-B., et al. (2002) Fertile hypomorphic ARGONAUTE (ago1) mutants impaired in post-transcriptional gene silencing and virus resistance. The Plant Cell 14, 629-639
27. Beclin, C., et al. (2002) A branched pathway for transgene-induced RNA silencing in plants. Current Biology 12, 684-688
28. Voinnet, O., et al. (1998) Systemic spread of sequence-specific transgene RNA degradation is initiated by localised introduction of ectopic promoterless DNA. Cell 95, 177-187
29. Vaistij, F.E., et al. (2002) Spreading of RNA targeting and DNA methylation in RNA silencing requires transcription of the target gene and a putative RNA-dependent RNA polymerase. Plant Cell 14, 857-867
30. Himber, C., et al. (2003) Transitivity-dependent and -independent cell-to-cell movement of RNA silencing. Embo J 22, 4523-4533
31. Szittya, G., et al. (2002) Short defective interfering RNAs of tombusviruses are not targeted but trigger post-transcriptional gene silencing against their helper virus. Plant Cell 14, 359-372
32. Muangsan, N., et al. (2004) Geminivirus VIGS of endogenous genes requires SGS2/SDE1 and SGS3 and defines a new branch in the genetic pathway for silencing in plants. Plant J 38, 1004-1014
33. Schwach, F., et al. (2005) An RNA-Dependent RNA Polymerase Prevents Meristem Invasion by Potato Virus X and Is Required for the Activity But Not the Production of a Systemic Silencing Signal. Plant Physiol
34. Bartel, D.P. (2004) MicroRNAs: genomics, biogenesis, mechanism, and function. Cell 116, 281-297
35. Xie, Z., et al. (2005) Expression of Arabidopsis MIRNA Genes. Plant Physiol
36. Jones-Rhoades, M.W., and Bartel, D.P. (2004) Computational Identification of Plant MicroRNAs and Their Targets, Including a Stress-Induced miRNA. Mol Cell 14, 787-799
37. Mallory, A.C., et al. (2005) MicroRNA-directed regulation of Arabidopsis AUXIN RESPONSE FACTOR17 is essential for proper development and modulates expression of early auxin response genes. Plant Cell 17, 1360-1375
38. Aukerman, M.J., and Sakai, H. (2003) Regulation of flowering time and floral organ identity by a MicroRNA and its APETALA2-like target genes. Plant Cell 15, 2730-2741
39. Baker, C.C., et al. (2005) The early extra petalsl mutant uncovers a role for microRNA miR164c in regulating petal number in Arabidopsis. Curr Biol 15, 303-315
40. Palatnik, J.F., et al. (2003) Control of leaf morphogenesis by microRNAs. Nature 425, 257-263
41. Juarez, M.T., et al. (2004) microRNA-mediated repression of rolled leaf1 specifies maize leaf polarity. Nature 428, 84-88
42. Kidner, C.A., and Martienssen, R.A. (2004) Spatially restricted microRNA directs leaf polarity through ARGONAUTE1. Nature 428, 81-84
43. Guo, H.S., et al. (2005) MicroRNA directs mRNA cleavage of the transcription factor NAC1 to downregulate auxin signals for arabidopsis lateral root development. Plant Cell 17, 1376-1386
44. Xie, Z., et al. (2003) Negative Feedback Regulation of Dicer-Likel in Arabidopsis by microRNA-Guided mRNA Degradation. Curr Biol 13, 784-789.
45. Vaucheret, H., et al. (2004) The action of ARGONAUTE1 in the miRNA pathway and its regulation by the miRNA pathway are crucial for plant development. Genes Dev 18, 1187-1197
46. Sunkar, R., and Zhu, J.K. (2004) Novel and stress-regulated microRNAs and other small RNAs from Arabidopsis. Plant Cell 16, 2001-2019
47. Bartel, B., and Bartel, D.P. (2003) MicroRNAs: at the root of plant development? Plant Physiol 132, 709-717
48. Parizotto, E.A., et al. (2004) In vivo investigation of the transcription, processing, endonucleolytic activity, and functional relevance of the spatial distribution of a plant miRNA. Genes Dev 18, 2237-2242
49. Wienholds, E., et al. (2005) MicroRNA expression in zebrafish embryonic development. Science 309, 310-311
50. Lee, Y., et al. (2004) MicroRNA genes are transcribed by RNA polymerase II. Embo J 23, 4051-4060
51. Kim, V.N. (2005) MicroRNA biogenesis: coordinated cropping and dicing. Nat Rev Mol Cell Biol 6, 376-385
52. Reinhart, B.J., et al. (2002) MicroRNAs in plants. Genes & Development 16, 1616-1626
53. Park, W., et al. (2002) CARPEL FACTORY, a Dicer homolog, and HEN1, a novel protein, act in microRNA metabolism in Arabidopsis thaliana. Curr Biol 12, 1484-1495
54. Kurihara, Y., and Watanabe, Y. (2004) Arabidopsis micro-RNA biogenesis through Dicer-like 1 protein functions. Proc Natl Acad Sci U S A 101, 12753-12758
55. Park, M.Y., et al. (2005) Nuclear processing and export of microRNAs in Arabidopsis. Proc Natl Acad Sci U S A 102, 3691-3696
56. Lund, E., et al. (2004) Nuclear export of microRNA precursors. Science 303, 95-98
57. Liu, Q., et al. (2003) R2D2, a bridge between the initiation and effector steps of the Drosophila RNAi pathway. Science 301, 1921-1925
58. Forstemann, K., et al. (2005) Normal microRNA maturation and germ-line stem cell maintenance requires Loquacious, a double-stranded RNA-binding domain protein. PLoS Biol 3, e236
59. Chendrimada, T.P., et al. (2005) TRBP recruits the Dicer complex to Ago2 for microRNA processing and gene silencing. Nature 436, 740-744
60. Lee, Y., et al. (2006) The role of PACT in the RNA silencing pathway. Embo J
61. Vazquez, F., et al. (2004) The nuclear dsRNA binding protein HYL1 is required for microRNA accumulation and plant development, but not posttranscriptional transgene silencing. Curr Biol 14, 346-351
62. Han, M.H., et al. (2004) The Arabidopsis double-stranded RNA-binding protein HYL1 plays a role in microRNA-mediated gene regulation. Proc Natl Acad Sci U S A 101, 1093-1098
63. Hiraguri, A., et al. (2005) Specific interactions between Dicer-like proteins and HYL1/DRB-family dsRNA-binding proteins in Arabidopsis thaliana. Plant Mol Biol 57, 173-188
64. Kurihara, Y., et al. (2006) The interaction between DCL1 and HYL1 is important for efficient and precise processing of pri-miRNA in plant microRNA biogenesis. Rna 12, 206-212
65. Dunoyer, P., et al. (2004) Probing the microRNA and small interfering RNA pathways with virus-encoded suppressors of RNA silencing. Plant Cell 16, 1235-1250
66. Schwarz, D.S., et al. (2003) Asymmetry in the assembly of the RNAi enzyme complex. Cell 115, 199-208
67. Yu, B., et al. (2005) Methylation as a crucial step in plant microRNA biogenesis. Science 307, 932-935
68. Yang, Z., et al. (2006) HEN1 recognizes 21-24 nt small RNA duplexes and deposits a methyl group onto the 2' OH of the 3' terminal nucleotide. Nucleic Acids Res 34,667-675
69. Ebhardt, H.A., et al. (2005) Extensive 3' modification of plant small RNAs is modulated by helper component-proteinase expression. Proc Natl Acad Sci U S A 102, 13398-13403
70. Shen, B., and Goodman, H.M. (2004) Uridine addition after microRNA-directed cleavage. Science 306, 997
71. Souret, F.F., et al. (2004) AtXRN4 degrades mRNA in Arabidopsis and its substrates include selected miRNA targets. Mol Cell 15, 173-183
72. Robb, G.B., et al. (2005) Specific and potent RNAi in the nucleus of human cells. Nat Struct Mol Biol 12, 133-137
73. Allen, E., et al. (2005) microRNA-directed phasing during trans-acting siRNA biogenesis in plants. Cell 121, 207-221
74. Bao, N., et al. (2004) MicroRNA binding sites in Arabidopsis class III HD-ZIP mRNAs are required for methylation of the template chromosome. Dev Cell 7, 653-662
75. Lu, C., et al. (2005) Elucidation of the small RNA component of the transcriptome. Science 309, 1567-1569
76. Vazquez, F., et al. (2004) Endogenous trans-acting siRNAs regulate the accumulation of Arabidopsis mRNAs. Mol Cell 16, 69-79
77. Peragine, A., et al. (2004) SGS3 and SGS2/SDE1/RDR6 are required for juvenile development and the production of trans-acting siRNAs in Arabidopsis. Genes Dev 18, 2368-2379
78. Yoshikawa, M., et al. (2005) A pathway for the biogenesis of trans-acting siRNAs in Arabidopsis. Genes Dev
79. Sessions, A., et al. (1997) ETTIN patterns the Arabidopsis floral meristem and reproductive organs. Development 124, 4481-4491
80. Hunter, C., et al. (2003) The Arabidopsis heterochronic gene ZIPPY is an ARGONAUTE family member. Curr Biol 13, 1734-1739
81. Borsani, O., et al. (2005) Endogenous siRNAs derived from a pair of natural cis-antisense transcripts regulate salt tolerance in Arabidopsis. Cell 123, 1279-1291
82. Yamada, K., et al. (2003) Empirical analysis of transcriptional activity in the Arabidopsis genome. Science 302, 842-846
83. Chen, J., et al. (2005) Genome-wide analysis of coordinate expression and evolution of human cis-encoded sense-antisense transcripts. Trends Genet 21, 326-329
84. Jackson, J.P., et al. (2004) Dimethylation of histone H3 lysine 9 is a critical mark for DNA methylation and gene silencing in Arabidopsis thaliana. Chromosoma 112, 308-315
85. Chan, S.W., et al. (2005) Gardening the genome: DNA methylation in Arabidopsis thaliana. Nat Rev Genet 6, 351-360
86. Matzke, M.A., and Birchler, J.A. (2005) RNAi-mediated pathways in the nucleus. Nat Rev Genet 6, 24-35
87. Jackson, J.P., et al. (2002) Control of CpNpG DNA methylation by the KRYPTONITE histone H3 methyltransferase. Nature 416, 556-560
88. Herr, A.J., et al. (2005) RNA polymerase IV directs silencing of endogenous DNA. Science 308, 118-120
89. Pontier, D., et al. (2005) Reinforcement of silencing at transposons and highly repeated sequences requires the concerted action of two distinct RNA polymerases IV in Arabidopsis. Genes Dev 19, 2030-2040
90. Onodera, Y., et al. (2005) Plant nuclear RNA polymerase IV mediates siRNA and DNA methylation-dependent heterochromatin formation. Cell 120, 613-622
91. Kanno, T., et al. (2005) Atypical RNA polymerase subunits required for RNA-directed DNA methylation. Nat Genet 37, 761-765
92. Kanno, T., et al. (2004) Involvement of putative SNF2 chromatin remodeling protein DRD1 in RNA-directed DNA methylation. Curr Biol 14, 801-805
93. Aufsatz, W., et al. (2002) HDA6, a putative histone deacetylase needed to enhance DNA methylation induced by double-stranded RNA. Embo J 21, 6832-6841
94. Ekwall, K. (2004) The RITS complex-A direct link between small RNA and heterochromatin. Mol Cell 13, 304-305
95. Jones, L., et al. (2001) RNA-directed transcriptional gene silencing in plants can be inherited independently of the RNA trigger and requires Metl for maintenance. Current Biology 11, 747-757
96. Schramke, V., et al. (2005) RNA-interference-directed chromatin modification coupled to RNA polymerase II transcription. Nature 435, 1275-1279
97. Weinberg, M.S., et al. (2006) The antisense strand of small interfering RNAs directs histone methylation and transcriptional gene silencing in human cells. Rna 12, 256-262
98. Volpe, T.A., et al. (2002) Regulation of heterochromatic silencing and histone H3 lysine-9 methylation by RNAi. Science 297, 1833-1837.
99. Liu, J., et al. (2004) siRNAs targeting an intronic transposon in the regulation of natural flowering behavior in Arabidopsis. Genes Dev 18, 2873-2878
100. Hirochika, H., et al. (2000) Silencing of retrotransposons in arabidopsis and reactivation by the ddml mutation. The Plant Cell 12, 357-368
101. Jeddeloh, J.A., et al. (1998) The DNA methylation locus DDM1 is required for maintenance of gene silencing in Arabidopsis. Genes & Development 12, 1714-1725
102. Ingouff, M., et al. (2005) Polycomb group genes control developmental timing of endosperm. Plant J 42, 663-674
103. Naumann, K., et al. (2005) Pivotal role of AtSUVH2 in heterochromatic histone methylation and gene silencing in Arabidopsis. Embo J 24, 1418-1429
104. Voinnet, O. (2005) Induction and suppression of RNA silencing: insights from viral infections. Nat Rev Genet 6, 206-220
105. Deleris, A., et al. (2006) Hierarchical action and inhibition of plant Dicer-like proteins in antiviral defense. Science 313, 68-71
106. Zhang, X., et al. (2006) Cucumber mosaic virus-encoded 2b suppressor inhibits Arabidopsis Argonautel cleavage activity to counter plant defense. Genes Dev 20, 3255-3268
107. Vargason, J.M., et al. (2003) Size selective recognition of siRNA by an RNA silencing suppressor. Cell 115, 799-811
108. Dangl, J.L., and Jones, J.D. (2001) Plant pathogens and integrated defence responses to infection. Nature 411, 826-833
109. Navarro, L., et al. (2004) The transcriptional innate immune response to flg-22. Interplay and overlap with Avr gene-dependent defense responses and bacterial pathogenesis. Plant Physiol 135, 1113-1128
110. Zipfel, C., et al. (2004) Bacterial disease resistance in Arabidopsis through flagellin perception. Nature 428, 764-767
111. Jones, J.D., and Dangl, J.L. (2006) The plant immune system. Nature 444, 323-329
112. Navarro, L., et al. (2006) A plant miRNA contributes to antibacterial resistance by repressing auxin signaling. Science 312, 436-439
113. Durrant, W.E., and Dong, X. (2004) Systemic acquired resistance. Annu Rev Phytopathol 42, 185-209
114. Xu, X., et al. (2006) Physical and functional interactions between pathogen-induced Arabidopsis WRKY18, WRKY40, and WRKY60 transcription factors. Plant Cell 18, 1310-1326
115. Zipfel, C., et al. (2006) Perception of the bacterial PAMP EF-Tu by the receptor EFR restricts Agrobacterium-mediated transformation. Cell 125, 749-760
116. Moissiard, G., et al. (2007) Transitivity in Arabidopsis can be primed, requires the redundant action of the antiviral Dicer-like 4 and Dicer-like 2, and is compromised by viral-encoded suppressor proteins. RNA 13, 1268-1278
117. Lopez-Solanilla, E., et al. (2004) HopPtoN is a Pseudomonas syringae Hrp (type III secretion system) cysteine protease effector that suppresses pathogen-induced necrosis associated with both compatible and incompatible plant interactions. Mol Microbiol 54, 353-365
118. Baumberger, N., et al. (2007) The Polerovirus Silencing SUppresor P0 Targets ARGONAUTE Proteins for Degradation. Current Biology 17, 1609-1614
119. Bortolamiol, D., et al. (2007) The polerovirus F Box Protein P0 Targets ARGONAUTE1 to Suppress RNA silencing. Current Biology 17, 1615-1621
120. Dunoyer, P., et al. (2007) Intra- and intercellular RNA interference in Arabidopsis thaliana requires components of the microRNA and heterochromatic silencing pathways. Nat Genet 39, 848-856
121. Chen, X. (2003) A MicroRNA as a Translational Repressor of APETALA2 in Arabidopsis Flower Development. Science
122. Gandikota, M., et al. (2007) The miRNA156/157 recognition element in the 3' UTR of the Arabidopsis SBP box gene SPL3 prevents early flowering by translational inhibition in seedlings. Plant J 49, 683-693
123. Fu, Z.Q., et al. (2007) A type III effector ADP-ribosylates RNA-binding proteins and quells plant immunity. Nature 447, 284-288
124. Schoning, J.C., et al. (2007) Auto-regulation of the circadian slave oscillator component AtGRP7 and regulation of its targets is impaired by a single RNA recognition motif point mutation. Plant J 52, 1119-1130
125. Taganov, K.D., et al. (2006) NF-kappaB-dependent induction of microRNA miR-146, an inhibitor targeted to signaling proteins of innate immune responses. Proc Natl Acad Sci U S A 103, 12481-12486
126. Rodriguez, A., et al. (2007) Requirement of bic/microRNA-155 for normal immune function. Science 316, 608-611
127. D'Acquisto, F., et al. (2002) Inhibition of Nuclear Factor Kappa B (NF-B):: An Emerging Theme in Anti-Inflammatory Therapies. Mol Interv 2, 22-35

## Claims

1. A method for modulating the miRNA pathway in plants and animals which method comprises
introducing into a plant or animal a nucleic acid construct comprising a constitutive or pathogen responsive promoter operatively linked to a pathogen-associated molecular pattern (PAMP)-responsive primary miRNA (pri-miRNA) sequence or a sequence that encodes a protein involved in miRNA biogenesis or activity.

2. The method of claim 1 wherein said pri-miRNA or said protein involved in miRNA biogenesis or activity provides enhanced pathogen resistance upon expression in said plant or animal.

3. The method of claim 1 wherein said pri-miRNA is selected from those in Table 2 herein.

4. The method of claim 3 wherein said pri-miRNA is other than miR393.

5. The method of claim 1 or 2 wherein said protein involved in miRNA biogenesis or activity is selected from the group consisting of DCL1, AGO1, SERRATE, and HEN1.

6. A plant or non-human animal prepared by the method of any of claims 1-5.

7. A nucleic acid construct as defined in any of claims 1-5.

8. A method to identify compounds useful in the selective modulation of the miRNA pathway in plants and animals which method comprises
exposing a plant or animal or cells of a plant or animal that have been modified to contain an expression system comprising control sequences for expression of an miRNA pathway component operably linked to a reporter sequence wherein said plant, animal or cells optionally further contain(s) an elicitor of said miRNA pathway component expression
to a candidate compound, and
detecting the presence or absence of expression of the reporter sequence,
whereby a compound that effects expression of the reporter sequence is identified as a compound useful in the selective modulation of the miRNA pathway in plants and animals.

9. A method to identify a gene useful in the selective modulation of the miRNA pathway in plants and animals which method comprises
mutagenizing a plant or animal or cells of a plant or animal that have been modified to contain an expression system comprising control sequences associated with expression of an miRNA pathway component operably linked to a reporter sequence wherein said plant, animal or cells optionally further contain(s) an elicitor of said miRNA pathway component expression, and
detecting the level of expression of the reporter sequence,
whereby a mutant that effects enhanced expression of the reporter sequence is identified as containing a gene mutation useful in the selective modulation of the miRNA pathway in plants and animals, and identifying the mutated gene.

10. A method to identify compounds useful in the selective modulation of the miRNA pathway in plants and animals which method comprises
exposing a plant or animal or cells of a plant or animal that have been modified to contain a constitutive expression system for a reporter sequence that can be silenced by an miRNA wherein said plant, animal or cells optionally further contain(s) an elicitor of said miRNA pathway component expression to a candidate compound, and
detecting the level of expression of the reporter sequence,
whereby a compound that decreases the level of expression of the reporter sequence is identified as a compound useful in the selective modulation of the miRNA pathway in plants and animals.

11. A method to identify a gene useful in the selective modulation of the miRNA pathway in plants and animals which method comprises
mutagenizing a plant or animal or cells of a plant or animal that have been modified to contain a constitutive expression for a reporter sequence that can be silenced by miRNA wherein said plant, animal or cells optionally further contain(s) an elicitor of said miRNA pathway component expression
to a candidate compound, and
detecting the level of expression of the reporter sequence,
whereby a compound that decreases the level of expression of the reporter sequence is identified as a compound useful in the selective modulation of the miRNA pathway in plants and animals.

12. A method to identify PAMP-responsive miRNAs precursors (pri-miRNA) which method comprises
isolating total RNA from said host; and
selecting RNA from total RNA isolated from a host modified to contain a protein that induces PAMP response that is expressed at higher levels in said host as compared to a host that has not been exposed to said protein and which hybridizes to complements of sequences upstream of identified stem loop structures in the genome.

13. An isolated PAMP-responsive pri-miRNA identified according to the method of claim 12.

14. The isolated PAMP-responsive pri-miRNA of claim 13 which is set forth in Table 2.

15. An isolated PAMP-responsive pri-miRNA of claim 14 which is other than miR393.

16. A method for conferring enhanced pathogen resistance on a plant or animal or plant or animal cell which comprises modifying said plant or animal or plant or animal cell to effect expression of the PAMP-responsive pri-miRNA of any of claims 12-15.

17. The method of claim 16 wherein said pri-miRNA is expressed under the operative control of a promoter which is activated on exposure of a plant or animal or plant or animal cell to a pathogen.

18. The method of claim 17 wherein said promoter is selected from the WRKY6 promoter and the PR1 promoter or other pathogen-responsive promoter including PAMP-responsive miRNA promoter.

19. The method of claim 18 wherein, one or more cis-acting regulatory element is disposed upstream of, or incorporated within, said promoter and miRNA expressing sequence.

20. The method of claim 19 wherein said cis-acting regulatory element is selected from the group consisting of a W box, an AuxRE element, an RY element, an FIM element, multiples of these elements and combinations thereof.

21. A bacterial silencing suppressor identified by a method disclosed herein.

22. A bacterial silencing suppressor of claim 21 selected from the group consisting of AvrPtoB, AvrPto, HopN1, HopH1, HopU1, HopT1-1 and HopY1.

23. A method for selectively modulating miRNA expression in a cell which comprises contacting said cell with the bacterial silencing suppressor of claim 21 or 22 or an expression system therefor.

24. A method to produce a desired protein in recombinant host cells which method comprises culturing cells that have been modified to express a bacterial silencing suppressor (Bss) and to express a nucleic acid construct for expression of a nucleotide sequence encoding the desired protein.

25. The method of claim 24 wherein the recombinant host cells are plant cells.

26. The method claim 24 or 25 wherein the Bss and desired protein are expressed as a fusion protein.

27. A recombinant expression system which comprises a nucleotide sequence encoding a fusion protein composed of a bacterial silencing suppressor (Bss) and a desired protein operably linked to control sequences that effect its expression in recombinant host cells.

28. The expression system of claim 27 wherein said control sequences are operable in plant cells.

29. The expression system of claim 26 or 27 wherein the desired protein is a protein that is therapeutically effective in animals.

30. An expression system which comprises a nucleotide sequence encoding a bacterial silencing suppressor operatively linked to heterologous control sequences.

31. A method of conferring resistance to pathogens in a plant which comprises selecting plants with components involved in miRNA biogenesis or activity that become resistant to the action of suppressors of silencing.

32. The method of claim 31 wherein said suppressor is a bacterial suppressor of silencing.

33. A plant selected according to the method of claim 31 or 32.

34. A method for specifically manipulating miRNA accumulation in order to alter physiological and developmental processes normally orchestrated by those molecules which comprises fusing a bacterial silencing suppressor Bss coding sequence to a tissue-specific plant promoter driving expression in roots, leaves, stem, inflorescences, optionally including patho-miR promoter elements to achieve spatial and temporal control of miRNA activity.
